# EUROPEAN PATENT SPECIFICATION

(11) **EP 2 790 732 B1**
(45) Date of publication and mention of the grant of the patent: **09.03.2016**
(21) Application number: 12818617.8
(22) Date of filing: 14.12.2012
(51) Int. Cl.: A61K 47/48, C07K 7/06, C07K 7/56, A61K 49/00, A61K 51/08, A61K 38/08

(54) **CLICKED SOMATOSTATIN CONJUGATED ANALOGS FOR BIOLOGICAL APPLICATIONS**
GEKLICKTE, SOMATOSTATINKONJUGIERTE ANALOGA FÜR BIOLOGISCHE ANWENDUNGEN
ANALOGUES CONJUGUÉS DE SOMATOSTATINE COUPÉS POUR DES APPLICATIONS BIOLOGIQUES

(30) Priority: 14.12.2011 EP 11193530
(43) Date of publication of application: 22.10.2014
(73) Proprietor: Centre National de la Recherche Scientifique (CNRS), 75794 Paris Cedex 16 (FR); Université de Bourgogne Dijon, 21000 Dijon (FR); Université de Cergy-Pontoise, 95011 Cergy-Pontoise Cedex (FR)
(72) Inventor: D'ADDONA, Debora, I-50019 Sesto Fiorentino (IT); BERNHARD, Claire, F-21000 Dijon (FR); ROVERO, Anna Maria, I-50127 Florence (IT); DENAT, Franck, F-21800 Quetigny (FR); CHOREV, Michael, Chesnut Hill Massachusetts 02467 (US)
(74) Representative: Cabinet Armengaud Aîné
(86) International application number: PCT/IB2012/057310
(87) International publication number: WO 2013/088395

(56) References cited:
- EP-A1- 1 598 366
- WO-A1-99/65508
- WO-A2-2010/004512
- D'ADDONA DEBORA ET AL: "Novel sst(5)-selective somatostatin dicarba-analogues: synthesis and conformation- affinity relationships", JOURNAL OF MEDICINAL CHEMISTRY, AMERICAN CHEMICAL SOCIETY, US, vol. 51, no. 3, 14 February 2008 (2008-02-14), pages 512-520, XP008104582, ISSN: 0022-2623, DOI: 10.1021/JM070886I [retrieved on 2008-01-23]
- FLAVIA BARRAGÁN ET AL: "Solid-phase synthesis and DNA binding studies of dichloroplatinum(ii) conjugates of dicarba analogues of octreotide as new anticancer drugs", CHEMICAL COMMUNICATIONS, no. 31, 1 January 2009 (2009-01-01), page 4705, XP055029959, ISSN: 1359-7345, DOI: 10.1039/b909698a
- ALESSANDRA DI CIANNI ET AL: "Novel Octreotide Dicarba-analogues with High Affinity and Different Selectivity for Somatostatin Receptors", JOURNAL OF MEDICINAL CHEMISTRY, vol. 53, no. 16, 26 August 2010 (2010-08-26), pages 6188-6197, XP055029962, ISSN: 0022-2623, DOI: 10.1021/jm1005868
- FLAVIA BARRAGÁN ET AL: "Photocontrolled DNA Binding of a Receptor-Targeted Organometallic Ruthenium(II) Complex", JOURNAL OF THE AMERICAN CHEMICAL SOCIETY, vol. 133, no. 35, 7 September 2011 (2011-09-07), pages 14098-14108, XP055029963, ISSN: 0002-7863, DOI: 10.1021/ja205235m
- MASUDA RYO ET AL: "Concise site-specific synthesis of DTPA-peptide conjugates: application to imaging probes for the chemokine receptor CXCR4.", BIOORGANIC & MEDICINAL CHEMISTRY 15 MAY 2011 LNKD- PUBMED:21524584, vol. 19, no. 10, 15 May 2011 (2011-05-15), pages 3216-3220, XP002677766, ISSN: 1464-3391
- RÖSCH FRANK ET AL: "Generator-based PET radiopharmaceuticals for molecular imaging of tumours: on the way to THERANOSTICS.", DALTON TRANSACTIONS (CAMBRIDGE, ENGLAND : 2003) 21 JUN 2011 LNKD- PUBMED:21445433, vol. 40, no. 23, 21 June 2011 (2011-06-21) , pages 6104-6111, XP002677767, ISSN: 1477-9234
- RIJKERS DIRK T S ET AL: "'Sulfo-click' for ligation as well as for site-specific conjugation with peptides, fluorophores, and metal chelators.", JOURNAL OF PEPTIDE SCIENCE : AN OFFICIAL PUBLICATION OF THE EUROPEAN PEPTIDE SOCIETY JAN 2010 LNKD- PUBMED:19924730, vol. 16, no. 1, January 2010 (2010-01), pages 1-5, XP002677768, ISSN: 1099-1387
- YIM CHENG-BIN ET AL: "Synthesis of DOTA-conjugated multimeric [Tyr3]octreotide peptides via a combination of Cu(I)-catalyzed "click" cycloaddition and thio acid/sulfonyl azide "sulfo-click" amidation and their in vivo evaluation.", JOURNAL OF MEDICINAL CHEMISTRY 27 MAY 2010 LNKD- PUBMED:20411957, vol. 53, no. 10, 27 May 2010 (2010-05-27), pages 3944-3953, XP002677769, ISSN: 1520-4804
- CARMEN WÄNGLER ET AL: "DOTA derivatives for site-specific biomolecule-modification via click chemistry: Synthesis and comparison of reaction characteristics", BIOORGANIC & MEDICINAL CHEMISTRY, PERGAMON, GB, [Online] vol. 19, no. 12, 15 June 2011 (2011-06-15) , pages 3864-3874, XP002677983, ISSN: 0968-0896, DOI: 10.1016/J.BMC.2010.12.047 Retrieved from the Internet: URL:http://www.sciencedirect.com/science/a rticle/pii/S0968089610011491> [retrieved on 2010-12-30]

## Description

The present invention is directed to somatostatin conjugated analogs and their use for biological applications, particularly for diagnosis, prognosis, monitoring disease activity, and evaluation of efficacy of therapeutic treatments. It also relates to the therapeutical applications thereof, advantageously for the curative treatments of tumors expressing somatostatin receptors.

### INTRODUCTION

The goal of the present invention is to provide new conjugated somatostatin analogs to fulfill the unmet needs of clinicians interested in more selective and stable agents that can be used as: 1) diagnostic tools in different imaging techniques, to efficiently and specifically target tumors. 2) cytotoxic or radiolabeled molecules for specific and efficacious targeted therapy of cancer.

### BACKGROUND / PRIOR ART / DRAWBACKS

The peptide hormone Somatostatin SRIF (for Somatotropin Release-Inhibiting Factor) has formula (A)

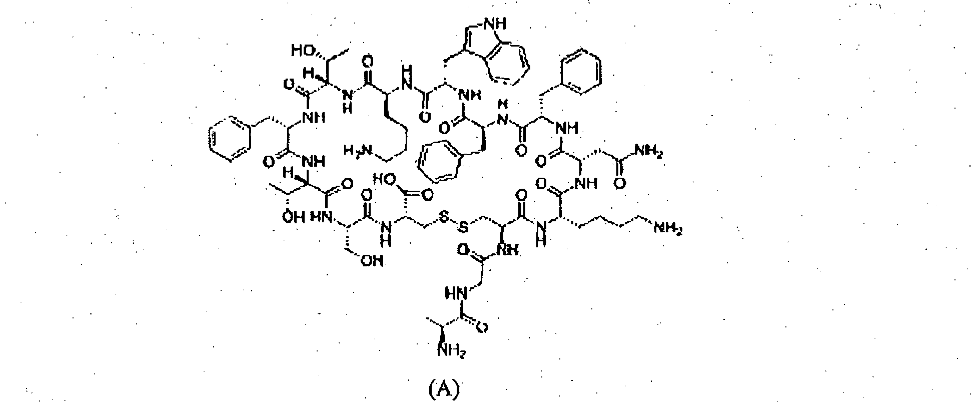

It appears in two active forms, one of 14 and the other of 28 amino acids. SRIFs are side chain-to-side chain disulflde-bridged cyclic peptides. They are predominantly produced by neurones and secretary cells in the central and peripheral nervous system and in the gastrointestinal tract. SRIFs are unique in their broad inhibitory effects on both endocrine secretion of hormones such as growth hormone (GH), insulin, glucagon, gastrin, cholecystokinin, Vasoactive Intestinal Peptide (VIP), and secretin, and exocrine secretion of fluids such as gastric acid, intestinal fluid, and pancreatic enzymes. In addition, the distribution of SRIFs in central nervous system and in the spinal cord makes them an important player in neuronal transmission.

The biological effects of SRIFs, all inhibitory in nature, are mediated by a family of structurally related, G-protein-coupled transmembrane receptors. These are classified into SRIF₁ receptor subtypes: sstr₂, sstr₃, and sstr₅, and SRIF₂ receptor subtypes: sstr₁ and sstr₄.

The unique pharmacological effects of SRIF-14 are derived from its universal high-affinity binding to all somatostatin-receptor subtypes. To overcome the short-lived presence of SRIF-14 in circulation (plasma half-life of < 3 min) many analogs consisting of cyclic peptides of 6 to 11 amino acids tethered by the disulfide bridge (Cys2-Cys7), have been developed in an attempt to stabilize the pharmacophoric β-turn region. Despite the large number of products developed up to now, only octreotide and lanreotide are approved for clinical use and pasereotide is in late phase of clinical development. These drugs are long acting, with circulating half-lives of about 90 min. However, their clinical use is limited, because they lack considerable endocrine selectivity. This family of drugs is much more efficacious than SRIF-14 in inhibiting the release of GH, glucagon, and insulin. In humans, long-term treatment with SRIF analogs is sometimes associated with hyperglycemia due to their inhibitory effects on insulin secretion.

In the field of diagnosis and therapy of Somatostatin-positive tumors, the SRIF analogs Octreoscan® and OctreoTher® are the mostly used monomodal diagnostic radiotracers. These are disulfide-bridged octapeptide somatostatin analogs of octreotide and Tyr-3-octreotide (TOC), respectively. These cyclopeptides are modified at the N-terminus with radiochelators. In particular, Octreoscan® is modified with DTPA-¹¹¹In (DTPA, diethylene triamine pentaacetic acid) and Octreother® is modified with DOTA-⁹⁰Y (DOTA, 2,2',2",2"'-(1,4,7,10-tetraazacyclododecane 1,4,7,10-tetrayl)tetraacetic acid).

Optimisation of these radiotracers for targeting tumoral cells requires increased bioavailability, better selectivity, and higher specificity. The following are some of the limitations presented by the current radiotracers that the invention is aiming to overcome:
The macrocyclic chelators should avoid: i) metal leaching into the body and consequently loss of selectivity/specific radioactivity; ii) high toxicity generally due to transchelation or transmetallation.

Moreover, the disulfide bridge present in SRIF analogs displays the following drawbacks: i) reduction of the disulfide by endogenous enzymes (i.e., by glutathione reductase and thioredoxin reductase); ii) cleavage by nucleophilic and basic agents; iii) interference with radiolabelling during the synthesis.

Intermolecular side chain-to-side chain cyclization is an established approach to achieve stabilization of specific conformations and a recognized strategy to improve resistance toward proteolytic degradation. Replacement of the disulfide bridge by end-to-end backbone cyclization as in the constraint analogs (i.e c[Phe-Pro-Phe-D-Trp-Lys-Thr]) were reported by Mattern and co workers. Despite a quite good affinity for sst sub-receptors they did not turn to be more potent or clinically useful somatostatin analogs.

Even if, the backbone cyclization substituting the disulfide bridge and proposed in a series of SRIF analogs, overcomes the side effect on hyperglycemia, a low affinity for some sst sub-receptors compared with octreotide makes this complex strategy not easily exploitable. Moreover, this cyclization linkage is still prone to endogenous cleavage (Afargan et al. Endocrinology. 2001, 142, 477-486 ; Conformationally constrained backbone cyclized somatostatin analogs Hornik, Vered; Seri-Levy, Alon; Gellerman, Gary; Gilon, Chalm From PCT Int. Appl. (1998), WO 9804583 A1 19980205).

Octreotide analogs cyclized via the dicarba-linkage bioisosteric to the disulfide bridge (managing only one single ring dimension) was already proposed by two of the inventors of the present patent application (D'Addona et al. .J. Med. Chem. 2008, 51, 512-520*, Dicarba-analogues of octreotide* WO2010004512A*).*

With these considerations in mind other side-chain to side-chain modifications shall be considered to introduce new bridging regions synthetically accessible (Le Chevalier Isaad A., Papini A. M., Chorev M., Rovero P. J. Pept. Sci. 2009; 15: 451-454) and less prone to oxidising and reducing attack.

The application of 1,2,3-triazoles has occurred only recently, following the discovery of regioselective Cu(I)-catalysed click chemistry in 2002. 1,2,3-triazoles show particular promise as amide bond isosteres, given their favourable pharmacophoric properties, excellent stability against isomerases and proteases and because of their accessible synthesis starting from a collection of synthetically available ω-alkynyl- and ω-azido- functionalised derivatives of chiral L and D amino acids (Formulae (B) and (C)) (A. Le Chevalier Isaad, F. Barbetti, P. Rovero, A.M. D'Ursi, M. Chelli, M. Chorev, A. M. Papini, Eur. J. Org. Chem., 2008, 31, 5308.)

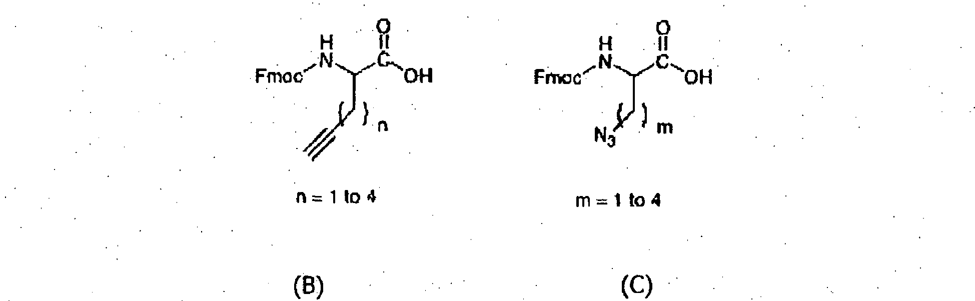

It is worth of note that, thanks to the different length of the alkynyl and azide amino acids side chains, the inventors of the present patent application were able to investigate not only the influence of the triazolyl moiety but also of the ring size on the bioactivity, selecting the right orientation, the number of methylene groups in amino-acid side chains to obtain more stable and specific analogues generating the optimal bioactive conformation.

Recently the incorporation of 1,2,3-triazoles was described: i) by Choi et al. as β-turn mimics into peptide nanotubes (W. J. Choi, Zhen-Dan Shi,a Karen M. Worthy, L. Bindu, Rajeshri G. Karki, M. C. Nicklaus, R. J. Fisher, T. R. Burke; Bioorganic & Medicinal Chemistry Letters-16-2006-5265-5269); ii) by Chorev and Papini for α-helical conformation stabilization (S. Cantel, A. Le Chevaller-Isaad, M. Scrima, J.J. Levy, R.D. DiMarchi, P. Rovero, J.A. Halperin, A.M. D'Ursi, A.M. Papini, M. Chorev; J. Org. Chem., 2008, 73, 5663-74); iii) by Jacobsen et al. to induce an 3₁₀-helical structure (Jacobsen Ø.,† Maekawa H., Ge N.-H, Göorbitz H. C., Rongved P., Ottersen O. P., Amiry-Moghaddam M., Klaveness J.; J. Org. Chem. 2011, 76, 1228-1238) iv) and in the meanwhile we were filing this patent appeared online disulfide bond mimetic analogues (Meldal M.; Angew. Chem. Int. Ed. Online DOI: 10-1002).

We argue that in order to enhance the stability and availability of new SRIF analogs *in vivo,* the use of 1,2,3-trlazole moieties leading to peptidomimetics reproducing native bioactive conformation (Scrima M.: Le Chevalier Isaad A.; Rovero P.; Papini A. M.; Chelll M.; Chorev, M.; D'ursi A. M. Eur. J. Org. Chem., 2010, 3, 446-457) have to be considered.

In view of the closest prior art recited above, the present inventors identified that remains a need for new somatostatin analogs that overcome the difficulties and problems reviewed above.

### Detailed description of the invention

The present invention enables to overcome the aforesaid problems by providing new conjugated somatostatin linear analogs of formula [I],
J" 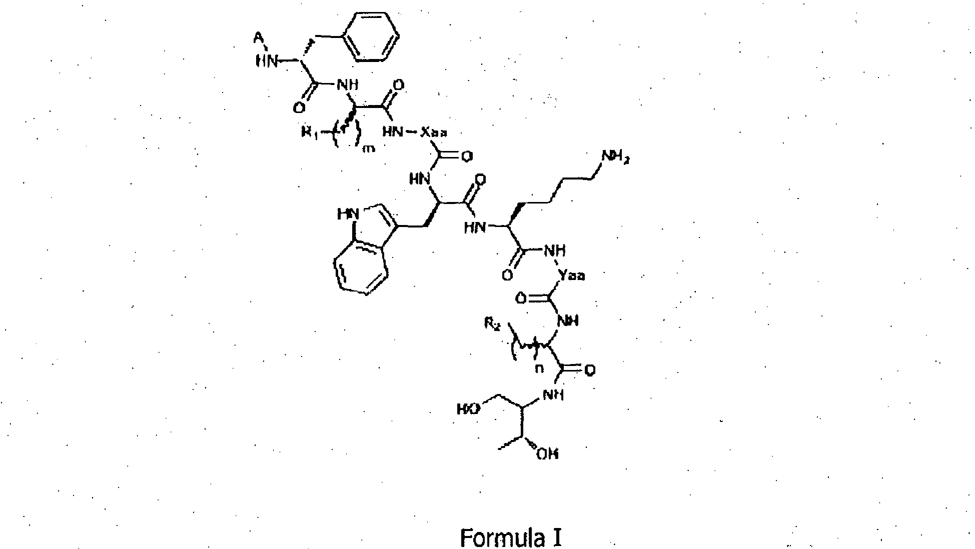
- wherein A- is H or TAG-B-,
   - wherein -B- is 0 or a spacer of formula C-D-E,
      o -C- and E are the same or different and are:
         ▪ a chain -(CH₂)ₗ-W where i ranges from 0 to 20 and can be preferably 0 and 1 and W is 0, a chain containing 1 to 6 (C=O) or (C=S) group(s) and/or one or more heteroatoms (N, O, S, P), and/or one or 1 to 3 aromatic moiety(ies) containing zero, one or more heteroatoms (N, O, S, P), ortho and/or meta and/or para substituted or a chain containing one to 12 amino acid moieties choosing among the 22 natural α-amino acids, β or γ amino acids residues, preferably lysine or threonine. C is advantageously selected from the group comprising 0, CH₂-, CH₂CH₂-, CH₂Ar-, CH₂CH₂Ar-, CH₂CONHCH₂CH₂-, CH₂CONHCH₂CH₂NHCH₂-,CH₂NHCOCH₂Ar-, CH2NHCOCHArNHCO CH₂-, CH₂NHCH₂ArOCH₂-. E is advantageously a group containing one amino acid moiety chosen among the 22 natural α-amino acids, β or γ amino acids residue or a group containing an unnatural amino acid modified on α amino or carboxyl group and/or in side chain position.
         ▪ a (PE_{G})ⱼ-W chain where j= 2, 4, 100, 300, 1000 and W is as described above
      o D being a group resulting from the coupling of two functional groups F and G present on the TAG and on the peptide respectively and defined as follows:
         D = thiourea generated from F = -NH₂ and G = -N=C=S (and vice versa), D = amide generated from F = NH₂ and G = COX (X = activated ester, Cl, anhydride,...) (and viceversa), D = thioether generated from F = maleimide- and G = -SH (and vice versa); D = 1,4-disubstituted [1,2,3]-triazole generated from F = -N=N⁺=N⁻ and G = -alkyne (and viceversa), D = imine generated from F = aldehyde and G = NH₂ (and vice versa). D = coupling function generated from F = norbornene or trans-cyclooctene and G = 1,2,4,5 tetrazine (and viceversa).
   - wherein TAG is
      - a chelating agent CA
         wherein CA-B is one of the formula 1 to 8.
         R and R' are groups capable of chelating a metal of interest, for either imaging purposes (Gd (MRI), ¹¹¹In or ⁶⁷Ga (SPECT), ⁶⁴Cu or ⁶⁸Ga (PET),...) or therapy (⁹⁰Y, ¹⁷⁷Lu, ⁶⁷Cu,...), preferentially COO⁻, COOH, P(O)(OH)Me or CONH₂. 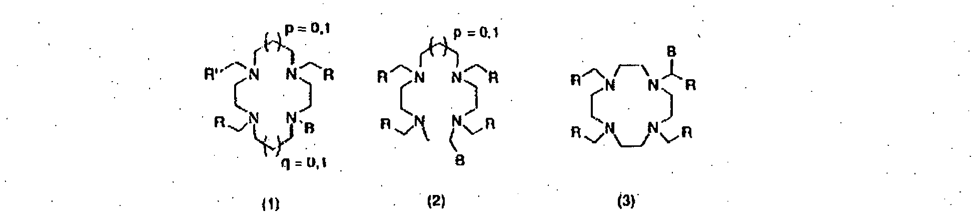 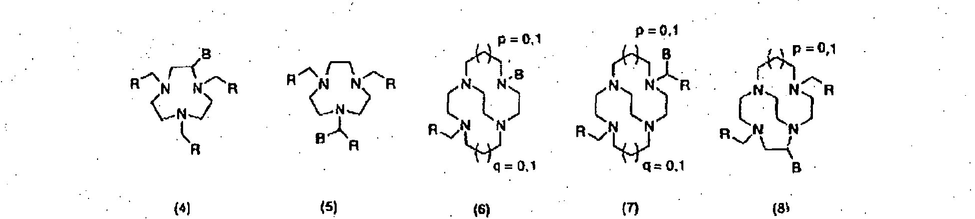
      - a fluorescent dye FD
         wherein FD-B is an organic fluorophore-B such as a Bodipy-B derivative, a Rhodamine-B derivative, a Fluorescein-B derivative, a Cyanine-B derivative, a Porphyrin-B derivative or a fluorescent complex of formulae (9) to (12) wherein J is a chromophore group and Ln is a Lanthanide chosen from Lanthanides giving high fluorescent Lanthanide complexes. 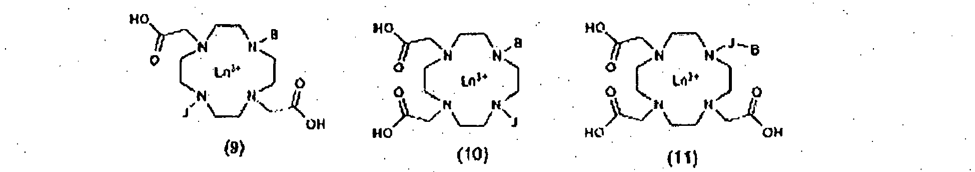 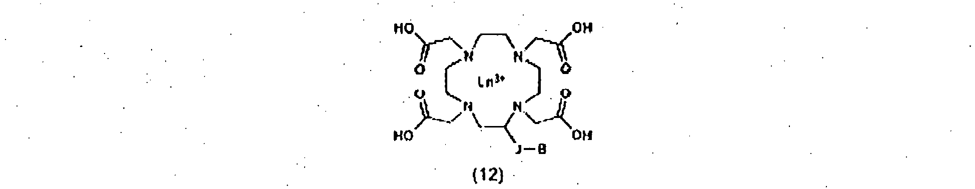
      - a bimodal agent BA comprising both a chelating agent CA and a fiuorophore FD coupled together particularly using an amino acid linker, for example BA-B being one of the formula 13 or 14. 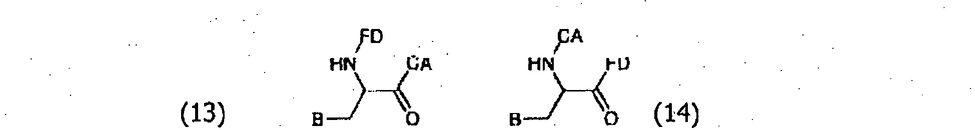 FD and B are the same as described previously.
      - a cytotoxic molecule CT
         wherein CT-B is a potent cytotoxic derivative of 2-pyrrolino-DOX (formula 15) or doxorubicin (DOX) of formula (16) capable to inhibit the growth of various tumors. 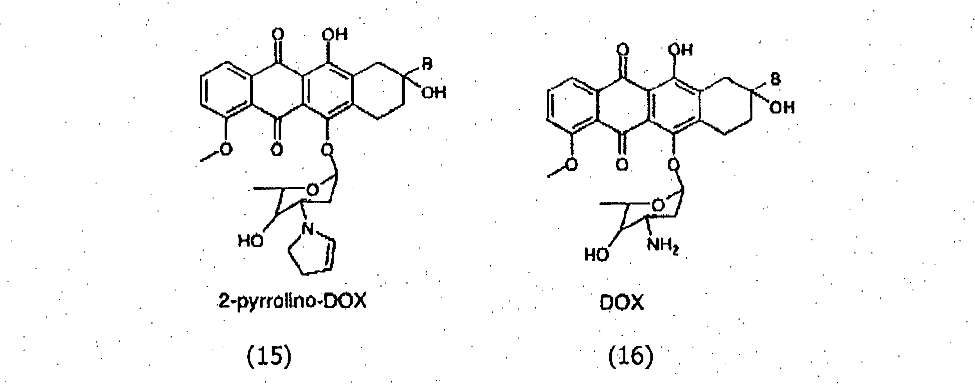
- wherein Xaa is all at least one of the 22 L or D amino acids or phenylalanine either unsubstituted or *ortho-* or para- substituted with the OR" group where, R" is linear or branched C1-C6 alkyl substituted ; otherwise Xaa is 1-Nal (Nal = naphthylalanine) or 2-Nal as such substituted on the 1 or 2 ring by OR" group where R" is H or linear or branched C1-C6 alkyl substituted or a C₆H₅-CH₂-.
- wherein Yaa is all 22 L or D amino acids or phenylalanine either unsubstituted or *ortho- or para-* substituted with the OR" group where, R" is linear or branched C1-C6 alkyl substituted or a threonine substituted with OR'" where R"' is H or a linear branched alkyl residue or a C₆H₅-CH₂- residue either unsubstituted or *ortho-* or *para-*substituted with the OR" group, R" being as above defined or a substituted C₆H₅-CH₂- or 1- or 2- naphthylmethyl- ; otherwise Yaa 1-Nal or 2-Nal as such unsubstituted or substituted on the 1 or 2 ring by -OCH₃ or C₆H₅-CH₂-O-.
- wherein R1 is C₁-C₄ alkynyl radical when R2 is C₁-C₄ azido radical and viceversa. m and n are the same or different and are 1 to 5.
In a group of analogues of the present invention, the compounds have formula (II), where R1 and R2 form an intramolecular [1, 2, 3] triazolyl bridge T. 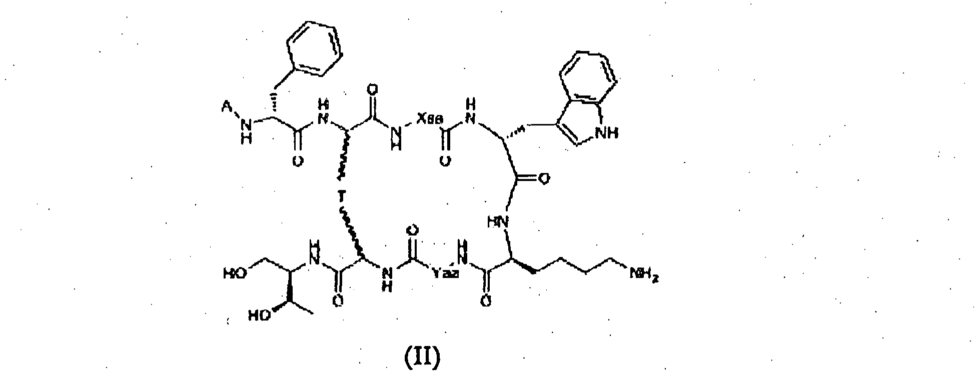 Wherein A, Xaa, Yaa are the same as described above. The orientation of the 1,4 disubstituted [1, 2, 3] triazolyl moiety depends on the position of ω-azido or ω-alkynyl aminoacids in the peptide chain, respectively i+5 and viceversa (formulae 1 and 2).

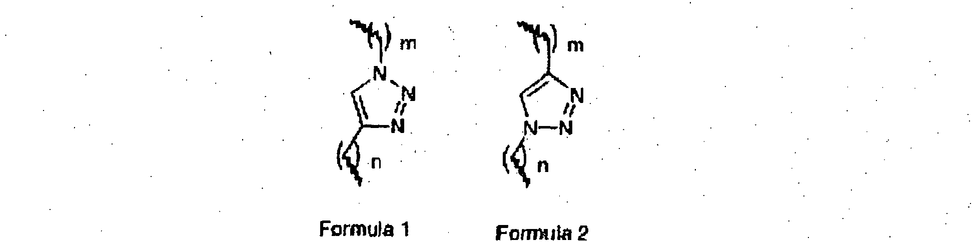

Analogs of formulae (I) and (II) wherein TAG-B is CA -B chelated with ¹¹¹In, ^{67/68}Ga or ⁶⁴Cu or FD-B or BA-B are more particularly useful for imaging.

Analogs of formulae (I) and (II) wherein TAG-B is CA-B and chelated with for example ⁹⁰Y, ¹⁷⁷Lu or ⁶⁷Cu are useful in therapy.

Analogs of formulae (I) and (II) wherein A is H can be useful in therapy.

Likewise, analogs of formulae (I) and (II) wherein TAG Is a cytotoxic molecule CT are of high value to inhibit the growth of various tumors.

The compounds of formulae (I) or (II) are advantageously prepared starting from known or easily prepared products.

The invention thus relates to a method for preparing the analog derivatives of formula (I):
- Solid Phase Peptide Synthesis (SPPS), comprising anchoring a peptide sequence to a derivatized chlorotrityl resin
- Recovering the crude peptide by treating the resin for example with trifluoroacetic acid
- Separating by precipitation for example ethyl ether
- Carrying out successive lyophilization
- Purifying the peptide using chromatographic techniques for example RP-HPLC

More particularly, the specific sequence by anchored to a derivatized chlorotrityl resin H-L-Thr(*t*-Bu)-ol-2-chlorotrityl resin. Upon completion of the synthesis the crude peptide were obtained by treating the resin with trifluoroacetic acid and separated by precipitation with ethyl ether and successive lyophilization. The peptide is finally purified using chromatographic technique; such as for example RP-HPLC.

The invention also relates to a method for preparing the derivative of formula (II) comprising
a) Synthetizing a linear heptapeptide following the Fmoc/tBu SPPS strategy with appropriate side chain protected aminoacids.
b) Cyclising the peptides linked to the resin by means of Cu(I)-catalyzed azide-alkyne 1,3 -dipolar Huisgen's cycloaddition to form regloselective 1,4- disubstituted - [1, 2, 3] triazolyl bridge
and either
c) adding the N-terminus aminoacid and the group A defined as described above in solid phase or alternatively following the solution strategy described in step c' to e'.
d) cleaving the conjugated 1,4- disubstituted- [1, 2, 3] triazolyl bridge containing peptides from the resin with an appropriate acid.
e) purifying by semipreparative RP-HPLC the crude 1,4-disubstituted - [1, 2, 3] triazolyl containing cyclic conjugated peptides.
or
c') adding the N-terminus amino acids and/or the group E-G, defined as described above; cleaving the peptide from the resin with an appropriate acid.
d') coupling the group TAG in solution to the 1,4-disubstituted - [1, 2, 3] triazolyl bridge containing peptides.
e') purifying by semipreparative RP-HPLC the crude 1,4-disubstituted - [1, 2, 3] triazolyl bridge containing peptides conjugated.

When A represents TAG-B, the method further comprises conjugating the resulting peptide to a tag derivative of formula (III)

TAG-C-F (III)

Wherein C and F are the same as described previously

The biological studies of the conjugated somatostatin linear analogs of formula [I] and 1,4-disubstituted [1,2,3]-triazolyl bridged somatostatin cyclopeptide analogs of formula [II] have shown these derivatives are of great interest in imaging and therapy of cancer.

They are able to deliver effectively, specifically and with minimal loss tags and therapeutic cytotoxic molecules to affected sites and organs to achieve optimal imaging or therapy of cancer.

Several features of the new cyclopeptide analogs contribute to confer to them a high selectivity and affinity for the different subtypes SST receptors, particularly, their bridging region with a bioisosteric heterocyclic moiety, the optimization of the length of the bridge and the location and orientation of the heterocyclic moiety in the bridge.

According to a first aspect, the invention thus relates to the use of the above defined compounds as radiotracers for imaging tumoral cells.

It particularly relates to a method of radio-isotoping imaging comprising the use of at least one compound according to formula (I) or (II) wherein A represents TAG-B.

In said method, the compound is administered by injection.

This method is particularly useful for SPECT/PET imaging and/or optical imaging. Mixing SPECT or PET with optical imaging enable detection by two imaging techniques and thus provide useful complementary diagnostic information.

According to a second aspect, due to their high binding affinity to sst 1-5 receptors, the compounds of the invention are therapeutic agents of interest as inhibitors for treating cancers, for example lymphoma, pancreatic, lung, prostate or breast cancer, or adenoma.

The invention thus also relates to pharmaceutical compositions comprising a therapeutically efficient amount of at least one compound of formulae (I) and (II) in combination with a pharmaceutically acceptable carrier.

The dosage in the pharmaceutical preparations will be easily determined by the one skilled in the art in view of the pathology to be treated. The doses per dosage unit will be chosen depending on the condition and age of the patient.

Examples disclosing the preparation of some conjugated peptides and chelating agents, according to the invention, are provided as follows to illustrate the purposes of the invention.

### 1) Synthesis of TAG where TAG = CA, containing three different groups F

### Example 1 : Synthesis of compound 1 where F is an acid function (1 step starting from the commercially available tri-tert-butyt 2,2',2"-(10-(2-((2-aminoethyl)amino)-2-oxoethyl)-1,4,7,10-tetraazacyclododecane-1,4,7-triyl)triacetate).

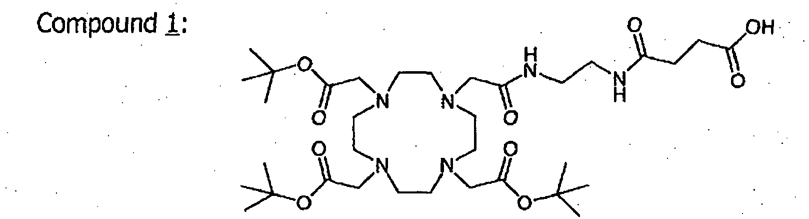

100 mg of succinic anhydride (1 mmol) were added to a solution of tri-tert-butyl 2,2',2"-(10-(2-((2-aminoethyl)amino)-2-oxoethyl)-1,4,7,10-tetraazacyclododecane-1,4,7-triyl)triacetate (620 mg, 1 mmol) in 10 mL of 1-4 Dioxane. The mixture was stirred at room temperature for 4 h. After evaporation of the solvent, the solid was taken in diethyl ether and filtered. The solvent was evaporated to give the compound 1 as a white foam (m = 680 mg, yield = 94%). ¹H NMR (300 MHz, CDCl₃, 300 K) δ(ppm): 1.41 (s, 27H, OC(CH₃)₃), 2.37-2.46 (m, 2H), 2.53-3.42 (m, 26H), 3.53-3.55 (m, 2H), 3.86-3.95 (m, 2H), 7.45 (bs, 1H, N*H*), 8.88 (bs, 1H, N*H*), 9.52 (bs, 1H, COO*H*).¹³C{¹H} NMR (75 MHz, CDCl₃, 300 K) δ (ppm): 28.3 (*9) (CH₃), 32.5, 32.7, 38.8, 39.4, 48.9 (*2), 50.8 (*2), 53.5 (*2), 53.7 (*2), 55.6, 56.1, 56.9 (*2) (CH₂), 81.8, 81.9 (*2) (C), 170.2 (*2), 170.4, 174.2, 177.8, 177.9 (C=O). MALDI-TOF: m/z = 715.27 [M+H]⁺, 737.944 [M+Na]⁺. Elemental analysis: C₃₄H₆₂N₆O₁₀• 2 H₂O• C₄H₈O₂. Calculated: C (54.40%), H (8.89%), N (10.42%). Obtained: C (54.32%), H (8.73%), N (10.02%).

### Example 2 : Synthesis of compound 2e where F is an isothiocyanate function (6 steps starting from the macrocycle 5-aminomethyl13aneN4 prepared according to the litterature (Rousselin, Y.; Sok, N.; Boschetti, F.; Guilard, R.; Denat, F. Eur. J. Org. Chem. 2010, 1688).

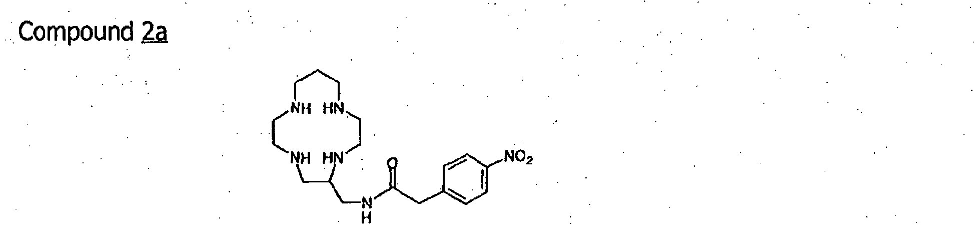

4.27 g of *N-*succinimidyl-4-nitrophenylacetate (1.53 mmol) were added to a solution of 5-aminomethyl13aneN4 (3.3g, 1.53 mmol) in dichloromethane (20 mL). The reaction mixture was stirred at room temperature for 2 h. Solvent was evaporated, the weak pink foam was dissolved in ethanol (10 mL) and a solution of HCl 35% (20 mL) was added. The precipitate was filtered and washed with ethanol (2*30 mL). The compound 2a (+ 4HCl) was obtained as a white solid, wich could be recrystallized in a mixture of water/ethanol. The resulting solid was dissolved rapidly in 15M NaOH solution until pH = 14. After extraction with chloroform (2*100 mL), the organic phase was dried over MgSO₄ and the solvent was evaporated to give a pink oil. This oil was taken in dichloromethane (10 mL), and upon addition of pentane (150 mL) a precipitate was formed slowly. The solution was left standing overnight to complete reprecipitation. The product was filtered, washed with pentane, and dried *in vacuo* to give 2a as a pink solid (m = 4.40 g, yield = 76%). ¹H NMR (300 MHz, CDCl₃, 300 K) δ (ppm): 1.48-1.76 (m, 2H, CH₂-β), 2.33-2.90 (m, 19H), 3.12-3.31 (m, 2H), 3.59 (s, 2H, CH₂Ar), 6.79 (bs, 1H, N*H*), 7.74 (d, 2H, *³*J = 8.6 Hz), 8.12 (d, 2H, *³*J = 8.6 Hz).¹³C{¹H} NMR (75 MHz, CDCl₃, 300 K) δ (ppm): 28.8 (CH₂-β), 43.4, 46.0, 47.6, 48.7, 48.9, 49.9, 50.5 55.6 (CH₂), 55.8 (CH), 65.9 (CH₂Ar), 123.9 (*2), 130.3 (*2) (CHₐᵣ), 143.1, 147.8 (Cₐᵣ), 169.2 (C=O). MALDI-TOF: *m*/*z* = 379.24 [M+H]⁺, 401.23 [M+Na]⁺. Elemental analysis: C₁₈H₃₀N₆O₃, 3 HCl, MeOH. Calculated: C (43.89%), H (7.17%), N (16.16%). Obtained: C (44.21%), H (7.01%), N (16.28%).

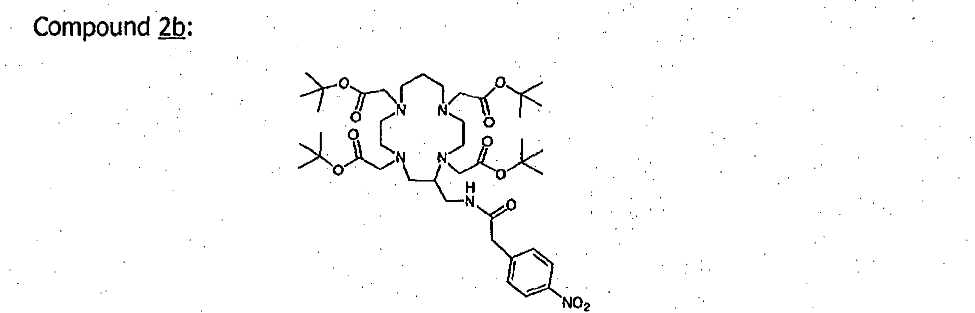

A solution of tertbutylbromoacetate (3.1 g, 16 mmol) was added to a solution of 2a (1.5 g, 3.9 mmol) and K₂CO₃ (3.7 g, 27 mmol) in acetonitrile (50 mL). The resulting mixture was heated at 45 °C overnight. After cooling, the solution was filtered on celite and the solvent was evaporated and the resulting oil was taken in ether. The mixture was filtered, the solvent evaporated, and the residue was purified to chromatography on aluminium oxide (eluent: CH₂Cl₂/MeOH 99:1) to give compound 2b as a yellow oil (1.6 g, yield = 48%). ¹H NMR (300 MHz, CDCl₃, 300 K) δ(ppm): 1.37-1.45 (m, 37H), 2.37-2.50 (m, 2H), 2.52-3.01 (m, 14H), 3.11-3.26 (m, 8H), 3.34-3.47 (m, 2H), 3.63 (s, 2H, CH₂Ar), 7.50 (d, 2H, *³*J = 8.6 Hz), 7.88 (bs, 1H, NH), 8.12 (d, 2H, *³*J = 8.6 Hz). ¹³C{¹H} NMR (75 MHz, CDCl₃, 300 K) δ(ppm): 25.2 (CH₂-β), 28.2 (*3), 28.4 (*9) (CH₃), 40.1, 43.5, 49.4, 49.9, 50.8, 52.3, 52.6, 53.1, 53.6, 55.1 (CH₂), 55.9 (CH), 56.8, 57.2, 57.9 (CH₂), 80.9 (*2), 81.1, 81.5 (C), 123.8 (*2), 130.4 (*2) (CHₐᵣ), 143.1, 147.0 (Cₐᵣ), 169.4, 171.0, 171.2, 171.5, 173.1 (C=O). MALDI-TOF: *m*/*z*= 857.31 [M+Na]⁺.

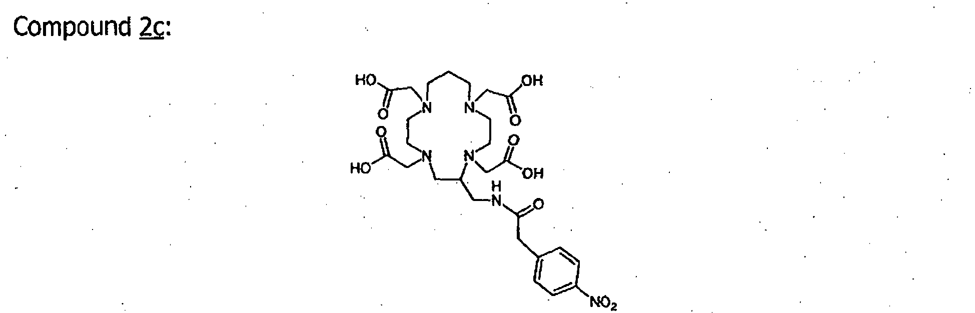

800 mg of compound 2b (95.8 mmol) were dissolved in 8 mL of HCl 35%. The mixture was stirred for 30 min at room temperature. The mixture was evaporated to dryness to give a brown solid, which was taken in 10 mL of acetone and stirred at room temperature overnight. The precipitate was filtered, washed with ethanol, acetone, ether and finally dried in vacuum. The compound 2c (+ 3HCl) was obtained as a white solid (m = 600 mg, yield = 95%). ¹H NMR (300 MHz, D₂O, 300 K) δ(ppm): 2.20-2.22 (m, 2H, CH₂-β), 2.85-3.96 (m, 25H), 4.02-4.24 (m, 2H), 7.47 (d, 2H, ³J = 8.65 Hz), 8.25 (d, 2H, ³J = 8.65 Hz). ESI-MS: *m*/*z* = 609.25 [M-H]⁻

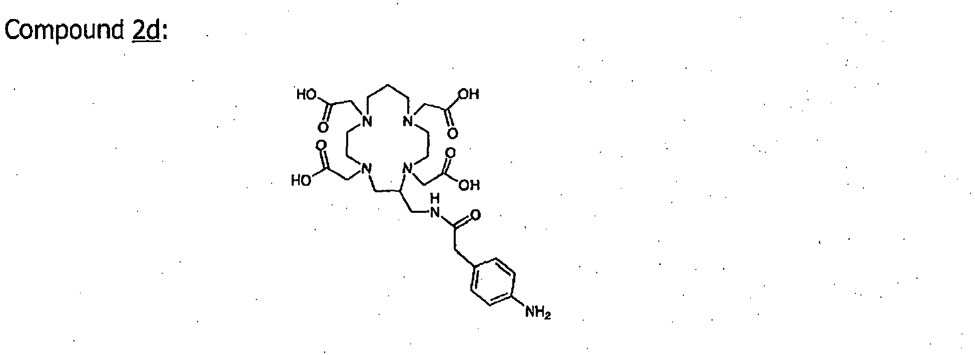

Compound 2c (100 mg, 0.14 mmol) was placed in 8 mL of water and 6 mg of 10 % Pd/C (5.58 µmol, 0.04 equivalent) was added under H₂. After consumption of the hydrogen, the suspension was eliminated by filtration on Clarcel^{®} and the solvent was evaporated to give 2d (+ 4 HCl) as a yellow solid (m = 90 mg, yield = 90%). ¹H NMR (300 MHz, D₂O, 300 K) δ(ppm): 2.06-2.10 (m, 2H, CH2-β), 2.78-4.02 (m, 27H), 7.17-7.37 (m, 4H). ESI-MS: *m*/*z* = 579.25 [M-H]⁻

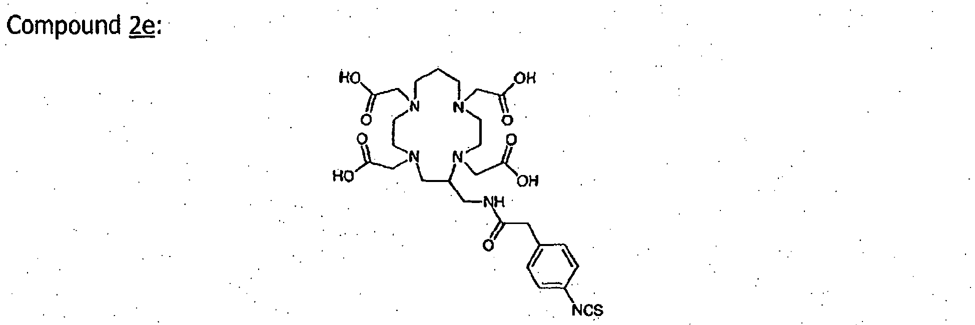

A solution of thiophosgene (30.4 µL, 0.41 mmol, 6 equivalents) in dichloromethane (2 mL) was added to a solution of 2d (50 mg, 68.8 µmol) in water (8 mL). After stirring vigorously for 2 h at room temperature, the resulting solution was washed with dichloromethane, the aqueous extracts were separated, and the solvent was evaporated. Compound 2e (+ 3HCl) was isolated as a yellow solid (m = 48 mg, yield = 98%). ¹H NMR (300 MHz, D₂O, 300 K) δ(ppm): 2.06-2.26 (m, 2H, CH₂-β), 2.76-4.26 (m, 27H), 7.01-7.52 (m, 4H). ESI-MS: *m*/*z* = 323.11 [(M+Na)/2]²⁺, 623.24 [M+H]⁺, 645.22 [M+Na]⁺

### Example 3: Synthesis of compound 3b where F is an alkyne function (2 steps starting from tri-tert-butyl 2,2',2"-(10-(2-((2-aminoethyl)amino)-2-oxoethyl)-1,4,7,10-tetraazacyclododecane-1,4,7-triyl)triacetate).

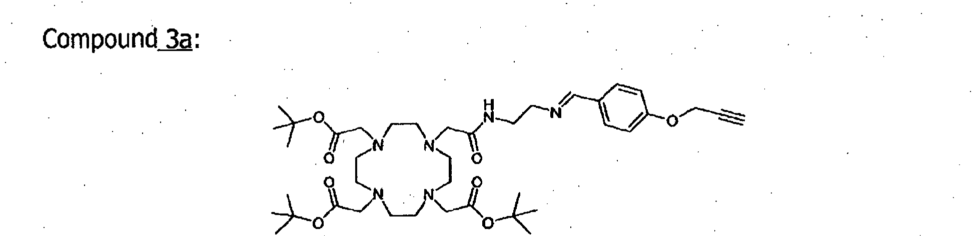

134 mg of 4-(prop-2-yn-1-yloxy)benzaldehyde (0.84 mmol) were added to a solution of tri-tert-butyl 2,2',2"-(10-(2-((2-aminoethyl)amino)-2-oxoethyl)-1,4,7,10-tetraaza cyclododecane-1,4,7-triyl)triacetate) (517 mg, 0.84 mmol) in ethanol (10 mL) and the mixture was stirred at room temperature for 12 h. The solvent was evaporated to dryness, and the residual oil was taken in pentane. After stirring during 12h, the insoluble impurities were removed by filtration. After evaporation of the solvent, the compound 3a was obtained as a white foam (m = 530 mg, yield = 84%). ¹H NMR (300 MHz, CDCl₃, 300 K) δ (ppm): 1.39 (s, 18H, OC(CH₃)₃), 1.41 (s, 9H, OC(CH₃)₃), 2.32-2.44 (m, 4H), 2.46 (t, 1H, *⁴*J = 2.4 Hz, CH*₂*-C≡C*H*), 2.50-2.79 (m, 12H), 2.90 (s, 2H), 3.06 (s, 4H), 3.20 (s, 2H), 3.40 (td, 2H, ³J =6.0 Hz, *³*J = 6.4 Hz, C*H₂*-CH₂-CH=N-), 3.61 (t, 2H, *³*J = 6.4 Hz, CH2-C*H₂*-CH=N-), 4.58 (d, 2H,*⁴*J = 2.4 Hz, C*H₂*-C≡CH), 6.85 (d, 2H, *³J* = 8.5 Hz), 7.54 (d, 2H, *³*J = 8.5 Hz), 8.09 (s, 1H, N=C*H-*), 8.63 (t, 1H, *³*J = 6.0 Hz, N*H*). ¹³C{¹H} NMR (150 MHz, CDCl₃, 300 K) δ (ppm): 27.2 (*9) (CH₃), 39.5, 50.9 (*2), 51.3 (*2), 52.6 (*2), 53.9 (*2), 54.8, 55.2 (*2), 55.3, 57.2, 59.4 (CH₂), 75.0 (CH), 77.1, 79.6, 79.8 (*2) (C), 113.8 (*2), 128.6 (*2) (CHₐᵣ), 128.9, 158.5 (Cₐᵣ), 160.4 (N=CH), 169.5, 169.6 (*2), 171.3 (C=O). MALDI-TOF: *m*/*z* = 779.43 [M+Na]⁺- HRMS-ESI: *m*/*z* = calculated for C₄₀H₆₄N₆O₈ + Na: 779.4678, obtained 779.467.

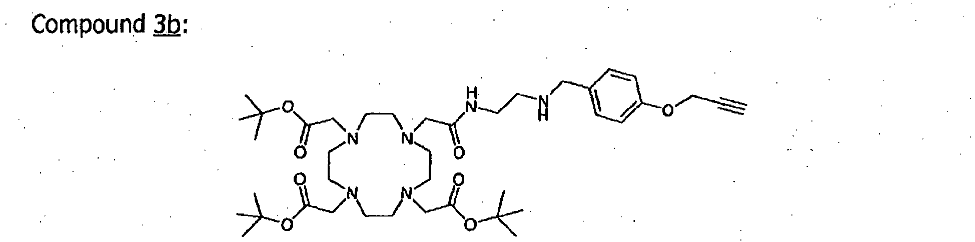

15 mg of NaBH₄ (0.38 mmol) were added to solution of 3a (0.9 g, 0.19 mmol) in ethanol (20 mL) to 0°C. The mixture was stirred overnight at room temperature. The solvent was evaporated, the resulting solid was dissolved in dichloromethane (20 mL). After filtration of the insoluble impurities, the solution was washed with a 1M NaOH solution (5 mL), dried over MgSO₄ and the solvent was evaporated to give 3b as a very hygroscopic white foam (m = 14 mg, yield = 80%).¹H NMR (300 MHz, CDCl₃, 300K) δ(ppm): 1.29-1.42 (m, 27H, OC(CH₃)₃), 1.97-3.15 (m, 25H), 2.46 (t, 1H, *⁴*J = 2.4 Hz, CH*₂*-C≡C*H*), 3.27-3.34 (m, 4H), 3.67 (bs, 2H), 4.60 (d, 2H, *⁴*J = 2.4 Hz, C*H₂-*C≡CH), 6.83 (d, 2H,³] = 8.5 Hz), 7.22 (d, 2H, *³*J = 8.5 Hz), 8.95 (bs, 1H, NH).¹³C{¹H} NMR (75 MHz, CDCl₃, 300K) δ(ppm): 27.9 (*3), 28.2 (*6) (CH₃), 39.0, 48.3, 50.0, 52.1, 52.6, 55.7, 55.8, 56.1, (16*CH₂), 75.3 (CH), 78.2, 79.6, 81.7, 81.8 (C), 113.8 (*2), 129.5 (*2) (CHₐᵣ), 133.9, 158.4 (Cₐᵣ), 171.8, 171.9 (*2), 172.4 (C=O). ESI-MS: *m*/*z* = 781.49 [M+Na]⁺. HRMS-ESI: *m*/*z* = calculated for C₄₀H₆₆N₆O₈ + Na: 781.4834, obtained 718.4822.

### 2) Synthesis of TAG where TAG = BA

### Exemple4: synthesis of compound 4e where F is an isothiocyanate function (5 steps starting from compound 4a).

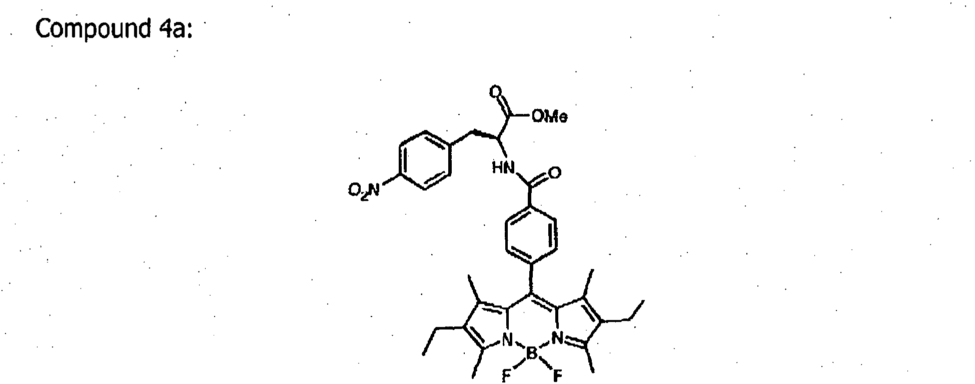

N-hydroxybenzotriazole (180 mg, 1.3 mmol), diisopropylethylamine (DIPEA) (340 mg, 2.6 memol), 1-(3-dimethylaminopropyl)-3-ethylcarbodiimide hydrochloride (EDCI) (250 mg, 1.3 mmol) and 4-nitrophenylalanine methylester hydrochloride (340 mg, 1.3 mmol) were successively added to a solution of 4-carboxyphenyl-1,3,5,7-tetramethyl-2,6-diethyl-4-bora-3a,4a-diaza-s-indacene (550 mg, 1.3 mmol) in dry DMF (30 mL), and the solution was stirred at room temperature. After total consumption of the starting material (8 hours) followed by TLC (AcOEt/hexane 6:4, Rf = 0.6), the solvent was evaporated. The solid obtained was washed with water (2* 30 mL) and extracted with dichloromethane (100 mL). The organic phase was dried over MagSO₄ and the solvent was evaporated to give a red oil. The crude product was purified by column chromatography on silica gel (AcOEt/hexane 1:1). Recrystallization in CH₂Cl₂/hexane gave pure 10 as red-green crystals (630 mg, 76%). ¹H NMR (300 MHz/CDl₃, 300K) δ(ppm): 0.93 (t, 6H, *³J* = 7.5 Hz), 1.23 (s, 6H), 2.28 (q, 4H, *³J =* 7.5 Hz), 2.51 (s, 6H), 3.34 (dd, 1H, *³J* = 13.9 Hz, 6.2 Hz), 3.45 (dd, 1H, J = 13.9 Hz, 5.1 Hz), 3.79 (s, 3H), 5.14 (ddd, 1H, *³J*= 7.0 Hz, *³J*= 6,2 Hz, *³J*= 5.1 Hz), 6.75 (d, 1H, *3J=* 7.0 Hz), 7.33 (d, 2H, *³J=* 8.7 Hz), 7.40 (d, 2H, *³J* = 8.2 Hz), 7.88 (d, 2H, *³J* = 8.3 Hz); 8.15 (d, 2H, *³J* = 8.7 Hz); ¹³C{¹H} NMR (75 MHz, CDCl₃, 300K) δ(ppm): 11.9, 12.6, 14.6, 17.1, 37.9, 52.8, 53.5, 123.8, 127.7, 129.1, 130.2, 130.3, 133.2, 133.7, 138.1, 138.5, 140.0, 143.7; 147.3, 154.4, 166.2, 171.6; ¹¹B NMR (128 MHz, CDCl₃, 300K): 0.78 ppm (t, *J_{B,F}*= 33.4 Hz); UV-Vis (CH₃CN), λ (nm) (ε, M⁻¹cm⁻¹): 523 (63000), 492 (21100), 378 (6800); HRMS-ESI *m*/*z:* calcd for C₃₄H₃₇BF₂N₄O₅ + H: 631.2903; found: 531.2897. Anal.Calcd for C₃₄H₃₇BF₂N₄O₅ + 0.3 CH₂Cl₂: C, 63.56; H, 5.82; N, 8.65; found: C, 63.44; H, 6.25; N, 8.63.

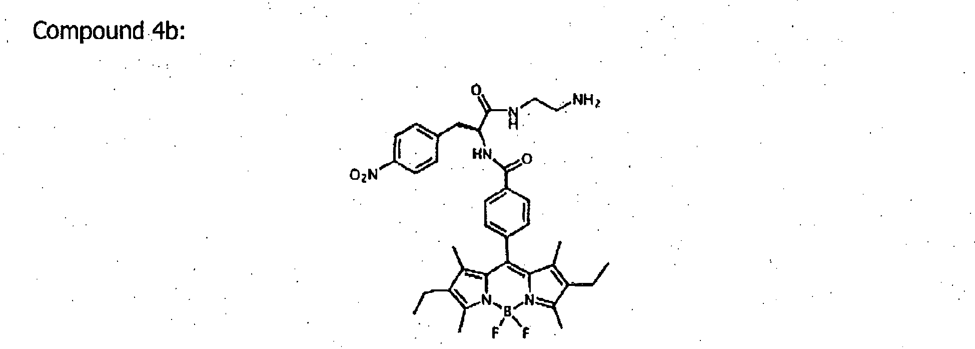

A solution of compound 4a (1.7 g, 2.7 mmol) and ethylenediamine (11.3 g, 0.18 mol) in 90 mL of methanol was stirred at 55°C for 48h. The solvent was evaporated, water was added (100 mL) and the product was extracted with dichloromethane (2*300 mL). The organic phase was dried over MgSO₄ and the solvent was evaporated. The crude product was washed with hexane (200 mL), and the solid obtained was purified by column chromatography on silica gel (CH₂Cl₂/MeOH/NH₄OH 80:18:2). Recrystallization in CH₂Cl₂/hexane gave pure 4b as red crystals (1.4 g, 79%). ¹H NMR (300 MHz, CDCl₃, 300K) δ(ppm): 0.95 (t, 6H, *³J* = 7.5 Hz), 1.22.(s, 6H), 2.27 (q, 4H, *³J* = 7.5 Hz), 2.51 (s, 6H), 2.64-2.81 (m, 2H), 3.18-3.36 (m, 2H), 4.87 (ddd, 1H, *³J* = 7.0 Hz, *³J* = 6.2 Hz, *3J=* 5.1 Hz), 6.36 (t, 1H, *³J* = 5.5 Hz, N*H*), 7.09 (d, 1H, *³J*= 7.0 Hz, N*H*), 7.38 (d,2H, *³J*= 8.2 Hz), 7.45 (d, 2H, *³J*= 8.5 Hz); 7.88 (d, 2H, *³J* = 8.2 Hz), 8.16 (d, 2H, *³J* = 8.5 Hz); ¹¹B NMR (128 MHz, CDCl₃, 300K): 0.77 ppm (t, *J_{B,F}* = 33.3 Hz); UV-Vis (CH₂Cl₂), λ (nm) (ε, M⁻¹ cm⁻¹): 528 (76500), 493 (23300), 379 (9650); ESI-MS: *m*/*z* = 639.32 [M-F]⁺, 659.33 [M+H]⁺; Anal.Calcd for C₃₅H₄₁BF₂N₆O₄+ 0.7 CH₂Cl₂: C, 59.72; H, 5.95; N, 11.70; found: C, 60.05; H, 5.79; N, 11.34.

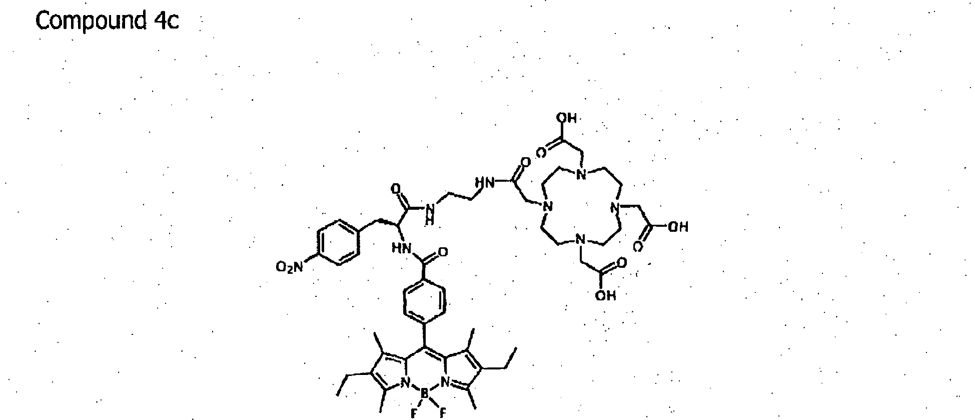

To a solution of compound 4b (230 mg, 0.34 mmol) and 170 µL of Et₃N (6 equivalents) in dry DMF (25 mL) was added a solution of DOTA-NHS ester (200 mg, 0.28 mmol) in dry DMF (5mL). The mixture was stirred at room temperature for 12h. Then the solvent was evaporated and the crude product was purified by column chromatography on silica gel (EtOH/NH₄OH 9:1). The solid obtained was washed with hexane (20 mL) and acetonitrile (20 mL). The compound 4c was isolated as a red solid (230 mg, 65%). ¹H NMR (600 MHz, MeOD, 330K) δ(ppm): 1.01 (t, 6H, *³J* = 7.5 Hz), 1.31 (s, 6H), 2.36 (q, 4H, *³J* = 7.5 Hz), 2.49 (s, 6H), 2.92-3.02 (m, 4H), 3.05-3.15 (m, 4H), 3.34-3.56 (m, 18H), 3.66-3.77 (m, 4H), 5.01-5.06 (m, 1H), 7.41 (d, 2H, *³J* = 8.2 Hz), 7.63 (d, 2H, *³J* = 8.5 Hz); 7.99 (d, 2H, *³J* = 8.2 Hz), 8.12 (d, 2H, *³J=* 8.5 Hz); ¹¹B NMR (128 MHz, MeOD, 300K): 0.72 ppm (t, *J_{B,F}*= 33.2 Hz); UV-Vis (DMF), λ (nm) (ε, M⁻¹cm⁻¹): 523 (64400), 491 (21400), 379 (6800); ESI-MS: *m*/*z* = 1067.49 [M+Na]⁺, 1089.47 [M+2Na-H]⁺, 1111.46 [M+3Na-2H]⁺, Anal.Calcd for C₅₁H₆₇BF₂N,₁₀O₁₁+ 6.5 H₂O, NH₄: C, 51.91; H, 7.17; N, 13.06; found: C, 51.75; H, 6.53; N, 12.38.

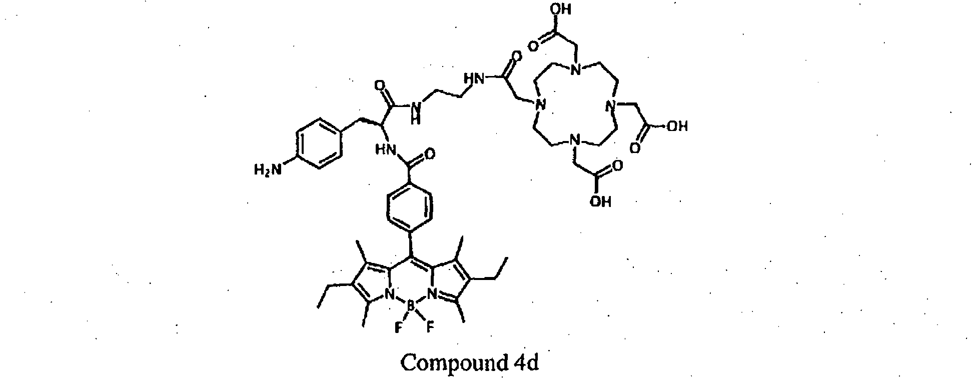

A suspension of compound 4c (50 mg, 47.8 µmol) and 10 % Pd/C (5mg, 19.2 µmol) in a mixture of water and ethanol (H₂O/EtOH, 90/10, 5 mL) was stirred under H₂. After consumption of hydrogen, the suspension was eliminated by filtration on Clarcel® and the solvent was evaporated. The solid obtained was washed with hexane (10 mL), to give compound 4d as a red solid (45 mg, 95%). ¹H NMR (300 MHz, MeOD, 300K) δ(ppm): 0.88 (t, 6H, *³J* = 7.5 Hz), 1.18 (s, 6H), 2.24 (q, 4H, *³J=* 7.5 Hz), 2.37 (s, 6H), 2.62-3.05 (m, 8H), 3.20-3.70 (m, 22H), 4.54-4.63 (m, 1H), 6.57 (d, 2H, *³J=* 8.2 Hz), 6.96 (d, 2H, *³J=* 8.5 Hz); 7.33 (d, 2H, *³J* = 8.2 Hz), 7.88 (d, 2H, *³J=* 8.5 Hz); ¹¹B NMR (128 MHz, MeOD, 300K): 0.71 ppm (t, *J_{B,F}*= 33.2 Hz); UV-Vis (DMF), λ (nm) (ε, M⁻¹cm⁻¹): 523 (60200), 491 (19200), 379 (5600); ESI-MS: *m*/*z* = 1051.43 [M+K-2H]⁻

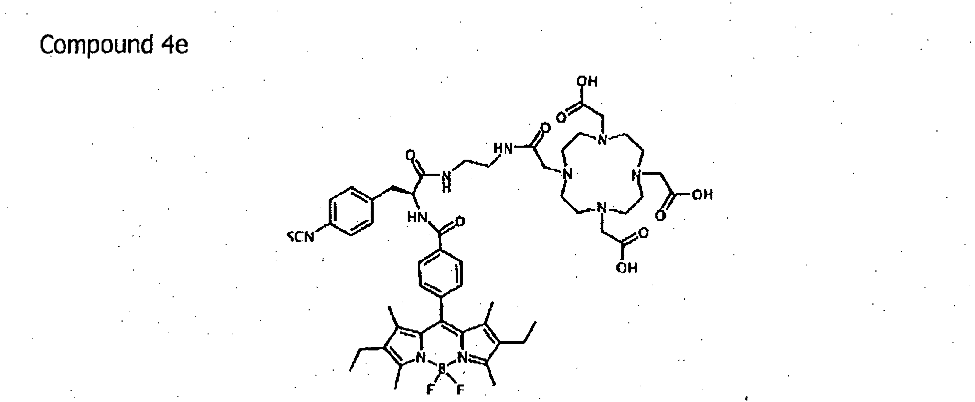

To a solution of 4d (15 mg, 14.7 µmol) In H₂O (5 mL) was added at room temperature a solution of thiophosgene (3.5 µL, 44.3 µmol) in chloroform (2 mL). The solution was stirred vigorously during 2h. The solvent was evaporated and the residue was lyophilized. The crude product was washed with CH₂Cl₂ (2 mL) and hexane (5 mL) to give 4e as a red solid (13.5 mg, 90%); UV-Vis (DMSO), λ (nm) (ε, M⁻¹cm⁻¹): 523 (43000), 492 (14400), 378 (4600); ESI-MS: *m*/*z* = 1077.45 [M+Na-2H]⁻; 1093.41 [M+K-2H]⁻; 1099.42 [M+2Na-3H]⁻

### 3) Synthesis of linear conjugated octapeptlde of Formula (I) where A is DOTA; m=n= 2; Xaa= -CH-CH₂-C₆H₅-OH; Yaa= -CH-CH(OH)-CH₃; R1= -N=N⁺=N; R2= alkynyl [DOTA-D-Phe-Abu(γ-N₃)-L-Tyr-D-Trp-L-Lys-L-Thr-L-amino-5-pentanoic-acid- L-Thr-ol].

The peptide was prepared in a Teflon reactor with a porous polystyrene septum, using the Fmoc/tBu SPPS strategy on pre-swollen H-L-Thr(*t*-Bu)-ol-2-chlorotrityl resin.

The coupling steps were carried out adding 2 eq. of protected amino acids, activated with HATU in case of unnatural synthetic amino acid (ω-alkynyl and ω-azido) and HOBt/HBTU (Hydroxybenzotriazoie/_2 - (1H - Benzotriazole - 1 - yl) - 1,1,3,3 - tetramethyluronium tetrafluoroborate) in other cases, and 4 eq. NMM In DMF, then stirred for 45 minutes and followed by Kaiser test monitoring. Fmoc- L-Lys-(Boc)-OH was coupled in position 5.

At the end of the synthesis the resin was treated with Trifluoroacetic acid/H₂O/1,2-Ethandithiol/Phenol (94:2:2:2) for 3 hours. This mixture allowed to cleave the peptide from the resin and simultaneously deprotecting all acid sensitive amino acid side-chains protecting groups. The solution was concentrated, the peptide was precipitated with Et₂O, filtered, dissolved in water and lyophilized. Analysis by RP-HPLC using Kinetex™ 2.6 µm C18 100 Å LC Column 150 x 3 mm, method 10-60% of B in A for 5 min (A = 0.1% TFA in H₂O, B = 0.1% TFA in CH₃CN) of the crude peptide has shown the presence of the linear 95% pure linear conjugated peptide at Rt= 4.09 min [M+H]⁺ 1452.51.

### Example 5: Synthesis of 1,4-disubstituted-[1,2;3]triazolyl bridge containing conjugate octapeptide formula II were A is DOTA; m=n= 2; Xaa= -CH-CH₂-C₆H₅-OH; Yaa= -CH-CH(OH)-CH₃; T= 1/4-disubstituted-[1/2/3]triazol (formula I).

### On resin strategy

Parent linear heptapeptide was synthesized as described above accordingly to the Fmoc/tBu SPPS strategy.

The on-resin linear heptapeptide was subjected to cyclization step. The cyclization of the peptide was carried out on the peptide linked to the resin by CuI-catalyzed azide-alkyne 1,3-dipolar Hulsgen's cycloaddition to form regioselective 1,4-disubstituted-[1,2,3]triazolyl bridge.

The peptidyl resin (250 mg) was swollen for 2 hours in DMC/MeOH 1/1. CuI (0.5 eq.) and DIPEA (40 eq.) were added under nitrogen fluxing into the suspended resin.

The suspension was left at r.t. for 15 hours. Conversion of the linear precursor into the 1,4-disubstituted-[1,2,3]triazolyl-containing peptide was monitored by microscale cleavage on the No terminal amino acid Fmoc-deprotected peptidyl resin. The crude cyclo-heptapeptide was analyzed by RP-HPLC using Kinetex™ 2.6 µm C18 100ÅLC Column 150 x 3 mm, method 10-60% of B in A for 5 min (A = 0.1% TFA in H₂O, B = 0.1% TFA in CH₃CN) showing complete conversion of linear precursor into cyclic one (Rt of cyclo-heptapeptide = 3.69 minutes).

All resin amount was then treated with 20% piperidine in DMF and the Fmoc group of the azido amino acid was removed. Subsequently 2 eq, of Fmoc- D-Phe-OH in DMF, 2 eg. HOBt/HBTU (Hydroxybenzotriazol/ 2 - (1H - Benzotriazol - 1 - yl) - 1,1,3,3 - tetramethyluronium tetrafluoroborate) and 4 eq. NMM were added to the resin. Fmoc group of the Nα terminal amino acid D-Phe was removed and 2 eq. of DOTA-tris-(^{t}Bu)-ester (CheMatech) in DMF, 2 eq. of HATU (*O*-(7-Azabenzotriazol-1-yl)-*N,N,N',N'*,-tetramethyluronium hexafluorophosphate) and 4 eq. of NMM were added to the resin. The 1,4-disubstituted=[1,2,3]triazoiyl-containing conjugated-octapeptide was cleaved as described above with the simultaneous deprotection of ^{t}Bu groups of the DOTA moiety.

The crude peptide was dissolved in water and lyophilized, then purified by semi-preparative RP-HPLC 20-50% of B in A for 20 min (A = 0.1% TFA in H₂O, B = 0.1% TFA in CH₃CN). Analysis of purified peptide with Kinetex™ 2.6 µm C18 100 Å LC Column 150 x 3 mm method 20-60% of B in A for 5 min A =0.1% TFA in H₂O, B = 0.1% TFA in CH₃CN) shows the peptide 1,4-disubstituted-[1,2,3]triazolyl-containing conjugated-octapeptide was >95% pure Rt = 3.11 minutes, [M+H]⁺ 1452.51.

Example 6. Synthesis of 1,4-disubstituted-[1,2,3]triazolyl bridge containing conjugate octapeptide formula II were A is DOTA; m=n= 2; Xaa= -CH-CH₂-C₆H₅-OH; Yaa= -CH-CH(OH)-CH₃; T= 1,4-disubstituted-[1,2,3]triazolyl group.

### In solution strategy

Synthesis of parent linear octapeptide was carried out as described in Example 5 anchoring at position 5 Fmoc-L-Lys(Dde).

The Fmoc Nα protecting group of D-Phe-OH was deprotected in 20% piperidine In DMF and the linear peptide was cleaved from the resin as described above to obtain D-Phe-Abu(γ-N₃)-L-Tyr-D-Trp-L-Lys(Dde)-L-Thr-L-amino-5-pentanoic-acid- L-Thr(ol).

Heterodetic cyclooctapeptide was generated in solution by intramolecular Cu(I)-catalyzed azido-alkyne 1,3-dipolar Huisgen's cycloaddition, in tBuOH/H₂O as solvent mixture, in the presence of 5 eq. of ascorbic acid and 5 eq. of Cu₂SO₄ generating *in situ* Cu(I). The crude cyclooctapeptide was analyzed by RP-HPLC (Kinetex™ 2.6 µm C18 100 A LC Column 150 x 3 mm, method 20-60% of B in A for 5 min A = 0.1% TFA in H₂O, B = 0.1% TFA in CH₃CN), showing complete conversion of the linear precursors (Rt shift from linear 4.0 minutes to heterodetic cyclootapeptide 3.5 minutes).

The DOTA chelating group was anchored using 4 eq. of DOTA, 5 eq. of NHS, 5 eq. of EDCI and 8 eq. of DIPEA in a mixture of water/DMF. The solvent was evaporated and methanol was added to the crude. The suspension was centrifuged and the solid discarded. The peptide dissolved in methanol was precipitated using Et₂O. Finally the Dde-protecting group on Lys was removed dissolving the peptide into 2% hydrazine hydrate in DMF. The peptide was then precipitated using Et₂O.

The crude heterodetic conjugated-cyclooctapeptide was dissolved in water and lyophilized, then purified by semi-preparative RP-HPLC 20-50% of B In A for 20 min (A = 0.1% TFA in H₂O, B = 0.1% TFA in CH₃CN). Analysis of purified peptide with Kinetex™ 2.6 µm C18 100 Å LC Column 150 x 3 mm method 20-60% of B in A for 5 min A = 0.1% TFA in H₂O, B = 0.1% TFA in CH₃CN) shows the peptide 1,4-disubstituted-[1,2,3]triazolyl-containing conjugated-octapeptide was >95% pure Rt = 3.11, minutes, [M+H]⁺ 1452.51.

### Example 7: ¹¹¹In-Radiolabeling of linear conjugated octapeptide of Formula (I) where A is DOTA; m=n= 2; Xaa= -CH-CH₂-C₆H₅-OH; Yaa= -CH-CH(OH)-CH₃; R1= - N=N⁺=N⁻; R2= alkynyl

20 MBq of ¹¹¹InCl₃ ([¹¹¹In]indium chloride (¹¹¹InCl_{3,} 370 MBq.mL⁻¹ in 0.05 N HCl) purchased from Perkin Elmer) were added to 12 µg of the DOTA-peptide In 0.1 M ammonium acetate buffer, pH 5.7, to reach a buffer / HCl (from ¹¹¹InCl₃ solution) ratio of 1.5:1 resulting in a pH 5 solution (Specific activity 2.36 MBq/nmol). The reaction was achieved in 1 hour at +75 °C and gave a radiolabellng yield of 96 % before purification, determined by radio-HPLC. HPLC analyses were performed on a Kinetex™ column 2.6 µm C18 100 Å LC Column 2.10 x 50 mm, coupled with a Flow-Count radio-HPLC Detection System (Bioscan®). Crude product was then purified through a Sep pack® C18 cartridge preconditioned as described in the literature. Free Indium was removed with water and the ¹¹¹In-DOTA-Peptide was eluted with a mixture of EtOH/PBS (7:3). The pure fractions were analyzed by radio-HPLC. ¹¹¹In-DOTA-peptide was obtained with a radiochemical purity > 98%.

### Example 8: ¹¹¹In-Radiolabeling of linear conjugated octapeptide of Formula (I) where A is DOTA; m=n= 2; Xaa= -CH-CH₂-C₆H₅-OH; Yaa= -CH-CH(OH)-CH₃; R1= alkynyl, R2= -N=N⁺=N⁻

20 MBq of ¹¹¹InCl₃ ([¹¹¹In]indium chloride (¹¹¹InCl₃, 370 MBq.mL⁻¹ In 0.05 N HCl) purchased from Perkin Elmer) were added to 7.35 µg of the DOTA-peptide in 0.1 M ammonium acetate buffer, pH 5.7, to reach a buffer / HCl (from ¹¹¹InCl₃ solution) ratio of 1.5:1 resulting in a pH 5 solution (Specific activity 5 MBq/nmol). The reaction was achieved in 1 hour at +75 °C and gave a radiolabeling yield of 98 % before purification, determined by radio-HPLC. HPLC analyses were performed on a Kfnetex™ column 2.6 µm C18 100 A LC Column 2.10 x 50 mm, coupled with a Flow-Count radio-HPLC Detection System (Bioscan®). Crude product was then purified through a Sep pack® C18 cartridge preconditioned as described in the literature. Free Indium was removed with water and the ¹¹¹In-DOTA-Peptide was eluted with a mixture of EtOH/PBS (7:3). The pure fractions were analyzed by radio-HPLC. ¹¹¹In-DOTA-peptide was obtained with a radiochemical purity > 99%.

## Claims

1. Conjugated somatostatin analogs of formula [1], 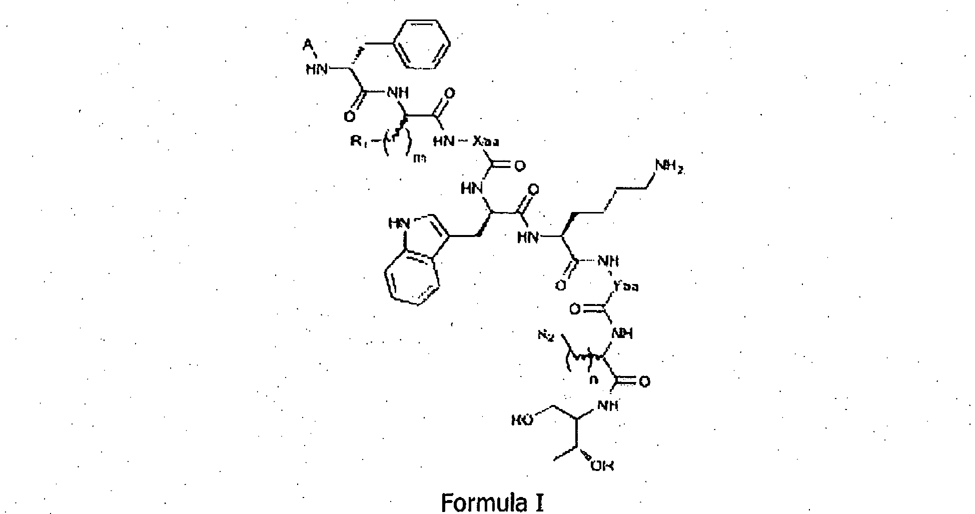
- wherein A- is H or TAG-B-,
• wherein -B- is 0 or a spacer of formula C-D-E,
∘ -C- and E are the same or different and are:
▪ a chain -(CH₂)ᵢ-W where i ranges from 0 to 20 and can be preferably 0 and 1 and W is 0, a chain containing 1 to 6 (C=O) or (C=S) group(s) and/or one or more heteroatoms (N, O, S, P), and/or one or 1 to 3 aromatic moiety(ies) containing zero, one or more heteroatoms (N, O, S, P), ortho and/or meta and/or para substituted or a chain containing one to 12 amino acid moieties choosing among the 22 natural α-amino acids, β or γ amino acids residues, preferably lysine or threonine.
▪ a (PE_{G})ⱼ-W chain where j= 2, 4, 100, 300, 1000 and W is as described above.
∘ D being a group resulting from the coupling of two functional groups F and G present on the TAG and on the peptide respectively and defined as follows:
D = thiourea generated from F = -NH₂ and G = -N=C=S (and vice versa), D = amide generated from F = NH₂ and G = COX (X = activated ester, Cl, anhydride,...) (and vlceversa), D = thloether generated from F = maleimide- and G = -SH (and vice versa); D = 1,4-disubstituted [1,2,3]-triazole generated from F = -N=N⁺=N⁻ and G = -alkyne (and viceversa), D = imine generated from F = aldehyde and G = NH₂ (and vice versa). D = coupling function generated from F = norbornene or trans-cyclooctene and G = 1,2,4,5 tetrazine (and viceversa).
• wherein TAG is
- a chelating agent CA
wherein CA-B is one of the formulae 1 to 8.
R and R' are groups capable of chelating a metal of interest, for either imaging purposes (Gd (MRI), ¹¹¹In or ⁶⁷Ga (ASPECT), ⁶⁴Cu or ⁶⁸Ga (PET),...) or therapy (⁹⁰Y, ¹⁷⁷Lu, ⁶⁷Cu,...), preferentially COO-, COOH, P(O)(OH)Me or CONH₂ 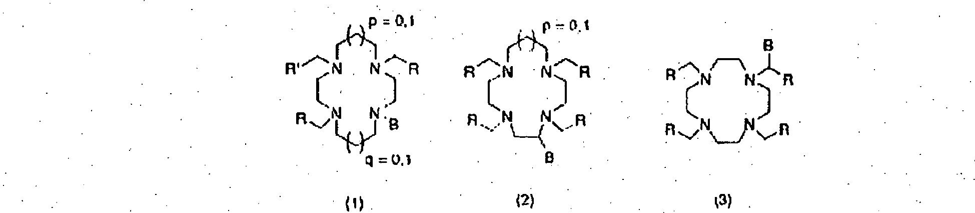 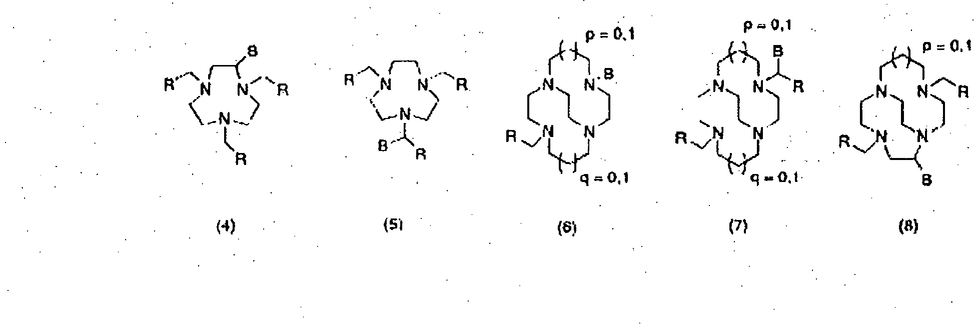
- a fluorescent dye FD
wherein FD-B is an organic fluorophore-B such as a Bodipy-B derivative, a Rhodamine-B derivative, a Fluorescein-B derivative, a Cyanine-B derivative, a Porphyrin-B derivative or a fluorescent complex of formulae (9) to (12) wherein J is a chromophore group and Ln is a Lanthanide chosen from Lanthanides giving high fluorescent Lanthanide complexes. 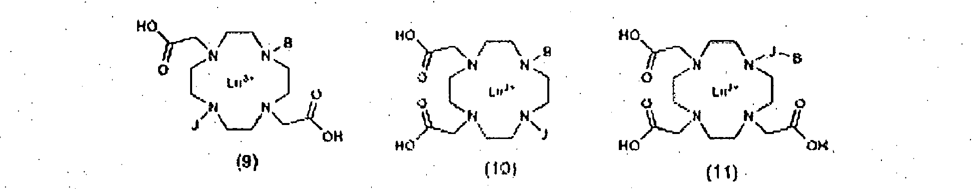 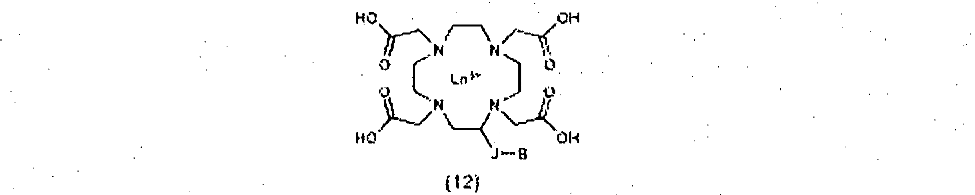
- a bimodal agent BA comprising both a chelating agent CA and a fluorophore FD coupled together particularly using an amino acid linker, for example BA-B being one of the formula 13 or 14 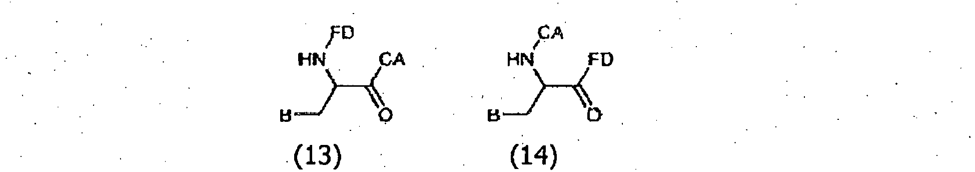 FD and B are the same as described previously.
- a cytotoxic molecule CT
wherein CT-B is a potent cytotoxic derivative of 2-pyrrolino-DOX (formula 15) or doxorubicin (DOX) of formula (16) capable to inhibit the growth of various tumors, 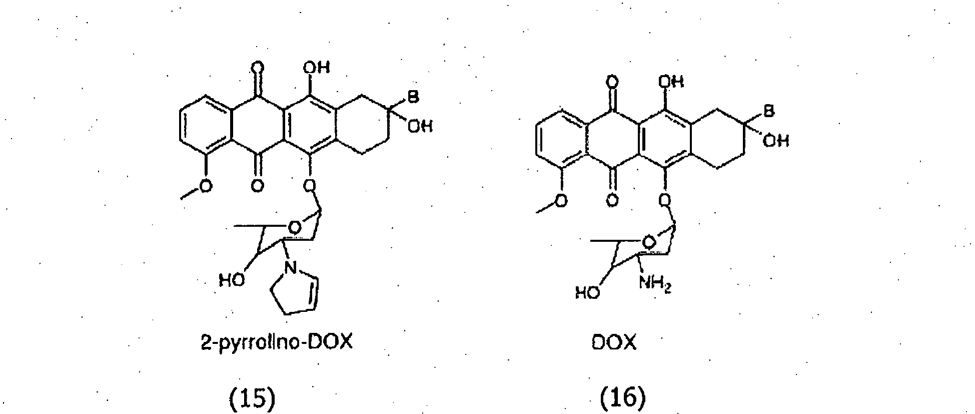
- wherein Xaa is all at least one of the 22 L or D amino acids or phenylalanin either unsubstituted or *ortho*- or *para*- substituted with the OR" group where, R" is linear or branched C1-C6 alkyl substituted ; otherwise Xaa is 1-Nal (Nal = naphthylalanine) or 2-Nal as such substituted on the 1 or 2 ring by OR" group where R" is H or linear or branched C1-C6 alkyl substituted or a C₆H₅-CH₂-,
- wherein Yaa is all 22 L, D amino acids or phenylalanin either unsubstituted or *ortho"-*or *para-* substituted with the OR" group where, R" is linear or branched C1-C6 alkyl substituted or a threonine substituted with OR"' where R"' is H or a linear branched alkyl residue or a C₆H₅-CH₂- residue either unsubstituted or *ortho-* or *para-*substituted with the OR" group, R" being as above defined or a substituted C₆H₅-CH₂- or 1- or 2- naphthylmethyl- ; otherwise Yaa 1-Nal or 2-Nal as such unsubstituted or substituted on the 1 or 2 ring by -OCH₃ or C₆H₅-CH₂-O- ;
- wherein R1 is C₂-C₄ alkynyl radical when R2 is C₁- C₄ azido radical and viceversa.
m and n are the same or different and are 1 to 5.

2. Conjugated somatostatin analogs of claim 1, wherein R1 and R2 form an intramolecular 1,4 disubstituted [1, 2, 3] triazolyl bridge T, the resulting compounds having formula (II) Wherein A, Xaa, Yaa are the same as described above. The orientation of the 1,4 disubstituted [1, 2, 3] triazolyl moiety depends on the position of ω-azido or ω-alkynyl aminoacids in the peptide chain, respectively i+5 and viceversa.

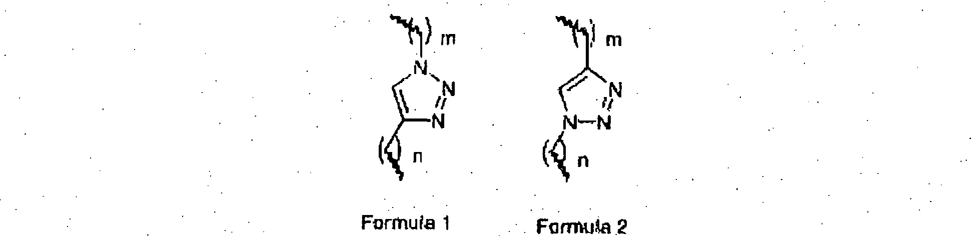

3. Conjugated somatostatin analogs of to claim 1 or 2, wherein:
- C is selected from the group comprising 0, CH₂-, CH₂CH₂-, CH₂Ar-, CH₂CH₂Ar-, CH₂CONHCH₂CH₂-, CH₂CONHCH₂CH₂NHCH₂-, CH₂NHCOCH₂Ar-, CH₂NHCOCHArNH COCH₂-, CH₂NHCH₂ArOCH₂-; or
- E is selected from a group containing one amino acid moiety chosen among the 22 natural α-amino acids, β or γ amino acids residues or a group containing an unnatural amino acid modified on α amino or carboxyl group and/or in side chain position.

4. Conjugated somatostatin analogs of anyone of claims 1 to 3, wherein A represents TAG-B.

5. Conjugated somatostatin analogs of anyone of claims 1 to 4 wherein TAG-B is CA -B chelated with ¹¹¹In, ^{68/69}Ga or ⁶⁴Cu or FD-B or BA-B for imaging.

6. Conjugated somatostatin analogs of anyone of claims 1 to 4, wherein TAG-B is CA-B and chelated with for example ⁹⁰Y, ¹⁷⁷Lu or ⁶⁷Cu for therapy.

7. Conjugated somatostatin analogs of anyone of claims 1 to 4, wherein TAG is a cytotoxic molecule CT.

8. A method for preparing the conjugated somatostatin analogs of formula (I) according to claim 1, by Solid Phase Peptide Synthesis (SPPS), comprising
- anchoring a peptidic sequence to a derivatized chlorotrityl resin
- recovering the crude peptide by treating the resin for example with trifluoroacetic acid
- separating by precipitation for example using ethyl ether
- carrying out successive lyophilization
- purifying the peptide using chromatographic technique for example RP-HPLC.

9. The method of claim 8, further comprising, for preparing the derivatives of formula (II),
a) Synthetizing a linear heptapeptide following the Fmoc/tBu SPPS strategy with appropriate side chain protected aminoacids.
b) Cyclising the peptides linked to the resin by means of Cu(I)-catalyzed azide-alkyne 1,3 -dipolar Huisgen's cycloaddition to form regioselective 1,4- disubstituted - [1, 2, 3] triazolyl bridge
and either
c) adding the N-terminus aminoacid and the group A defined as described above in solid phase or alternatively following the solution strategy describe in step c' to e'.
d) cleaving the conjugated 1,4- disubstituted- [1, 2, 3] triazolyl bridge containing peptides from the resin with an appropriate acid
e) purifying by semipreparative RP-HPLC the crude 1,4-disubstituted - [1, 2, 3] triazolyl containing cyclic conjugated peptides
or
c') adding the N-terminus amino acids and/or the group E-G, defined as described above; cleaving the peptide from the resin with an appropriate acid.
d') coupling the group TAG in solution to the 1,4-disubstituted - [1, 2, 3] triazolyl bridge containing peptides.
e') purifying by semipreparative RP-HPLC the crude 1,4-disubstituted - [1, 2, 3] triazolyl bridge containing peptides conjugated.

10. The method of claim 8 or 9, further comprising, when A represents TAG-B (or TAG-C-D-E), conjugating the resulting peptide to a tag derivative of formula (III)
TAG-C-F (III)
wherein C and Fare the same as described above.

11. Compounds according to claims 1 to 5 used for optical imaging.

12. Radiotracers according to claim 1 to 6 used for SPECT/PET imaging or for nuclear/optical imaging; or comprising at least one conjugated somatostatin analogs according to anyone of claims 1 to 6 for imaging tumoral cells.

13. A method of radioisotope Imaging comprising the use of at least one compound according to formula {I) or (II) wherein A represents TAG-B.

14. Pharmaceutical compositions comprising a therapeutically efficient amount of at least one conjugated somatostatin analog of formula (I) in combination with a pharmaceutically acceptable carrier.

15. The pharmaceutical compositions of claim 14, wherein said conjugated somatostatin analog(s) is (are) used for the treatment of cancer, in particular for the treatment of lymphoma, pancreatic, lung, prostate or breast cancer, or adenoma.

## Patentansprüche

1. Konjugierte Somatostatin-Analoga der Formel [I] 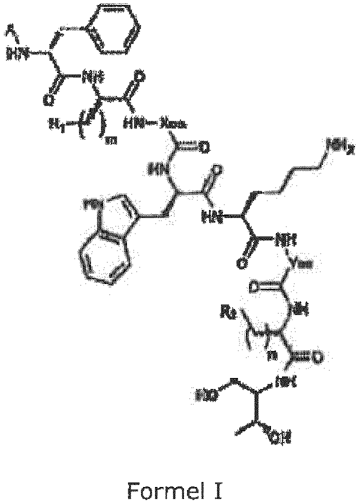
- wobei A- = H oder TAG-B- ist,
• wobei -B- = 0 oder ein Spacer einer Formel C-D-E ist,
o -C- und E gleich oder verschieden sind und Folgendes sind:
▪ eine Kette -(CH₂)ᵢ-W, wobei i im Bereich von 0 bis 20 liegt und bevorzugt 0 und 1 sein kann und W = 0 ist, eine Kette enthaltend 1 bis 6 (C=O) oder (C=S) Gruppe(n) und/oder ein oder mehrere Heteroatome (N, O, S, P) und/oder eine oder 1 bis 3 aromatische Struktureinheiten, die null, ein oder mehrere Heteroatome (N, O, S, P) enthalten, ortho-und/oder meta- und/oder para-substituiert, ist oder eine Kette enthaltend ein bis 12 Aminosäurereste ausgewählt aus den 22 natürlichen α-Aminosäuren, β- oder γ-Aminosäureresten, bevorzugt Lysin oder Threonin, ist,
▪ eine (PEG)ⱼ-W-Kette, wobei j = 2, 4, 100, 300, 1000 und W wie vorstehend beschrieben ist,
∘ wobei D eine Gruppe ist, die aus der Kopplung zweier funktionaler Gruppen F und G resultiert, die an dem TAG und an dem Peptid jeweils vorhanden sind, und wie folgt definiert ist:
D = Thioharnstoff, erzeugt aus F = -NH₂ und G = -N=C=S (und umgekehrt), D = Amid, erzeugt aus F = NH₂ und G = COX (X = aktivierter Ester, Cl, Anhydrid, ...) (und umgekehrt), D = Thioether, erzeugt aus F = Maleinimid- und G = -SH (und umgekehrt); D = 1,4-disubstituiertes [1,2,3]-Triazol, erzeugt aus F = -N = N⁺ = N⁻ und G = -Alkin (und umgekehrt), D = Imin, erzeugt aus F = Aldehyd und G = NH₂ (und umgekehrt), D = Kopplungsfunktion, erzeugt aus F = Norbornen oder trans-Cycloocten und G = 1,2,4,5-Tetrazin (und umgekehrt),
• wobei TAG Folgendes ist:
- ein Chelatbildner CA,
wobei CA-B eine der Formeln 1 bis 8 ist,
R und R' Gruppen sind, die fähig sind, ein Metall von Interesse zu chelatisieren, entweder zu bildgebenden Zwecken (Gd (MRI), ¹¹¹In oder ⁶⁷Ga (SPECT), ⁶⁴Cu oder ⁶⁸Ga (PET), ...) oder zur Therapie (⁹⁰Y, ¹⁷⁷Lu, ⁶⁷Cu, ...), bevorzugt COO⁻, COOH, P(O)(OH)Me oder CONH₂: 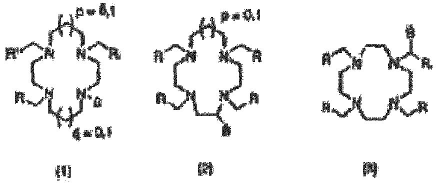 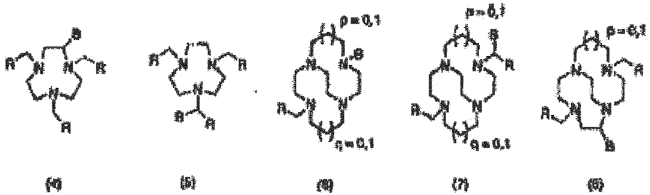
- ein Fluoreszenz-Farbstoff FD,
wobei FD-B ein organisches Fluorophor-B, wie ein Bodipy-B-Derivat, ein Rhodamin-B-Derivat, ein Fluorescein-B-Derivat, ein Cyanin-B-Derivat, ein Porphyrin-B-Derivat oder ein Fluoreszenz-Komplex der Formeln (9) bis (12) ist, wobei J eine chromophore Gruppe ist und Ln ein Lanthanid ist, das aus Lanthaniden, die hochfluoreszierende Lanthanid-Komplexe ergeben, ausgewählt ist: 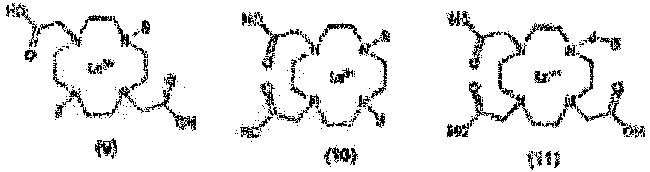 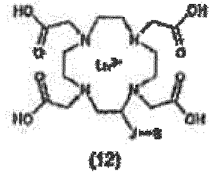
- ein bimodafes Mittel BA, enthaltend sowohl einen Chelatbildner CA als auch ein Fluorophor FD, zusammen gekoppelt insbesondere unter Verwendung eines Aminosäure-Linkers, wobei zum Beispiel BA-B eine der Formel 13 oder 14 ist: 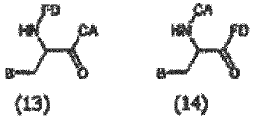 FD und B die gleichen wie vorstehend beschrieben sind,
- ein cytotoxisches Molekül CT,
wobei CT-B ein potentes cytotoxisches Derivat von 2-Pyrrolino-DOX (Formel 15) oder Doxorubicin (DOX) einer Formel (16) ist, das fähig ist, das Wachstum verschiedener Tumoren zu hemmen, 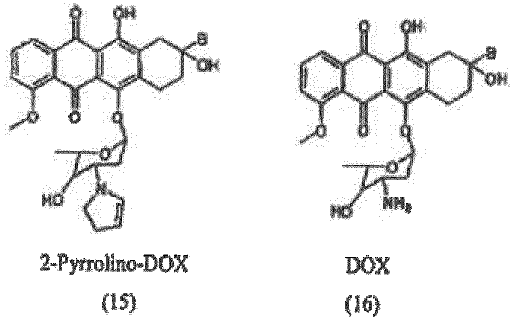
- wobei Xaa insgesamt wenigstens eine der 22 L- oder D-Aminosäuren oder Phenylalanin entweder unsubstituiert oder ortho- oder para-substituiert mit der OR"-Gruppe ist, wobei R" lineares oder verzweigtes substituiertes C₁-C₆-Alkyl ist; andernfalls Xaa = 1-Nal (Nal = Naphthylalanin) oder 2-Nal als solches substituiert an dem 1- oder 2-Ring durch eine OR"-Gruppe ist, wobei R" = H oder lineares oder verzweigtes substituiertes C₁-C₆-Alkyl oder ein C₆H₅-CH₂- ist,
- wobei Yaa insgesamt 22 L-, D-Aminosäuren oder Phenylalanin entweder unsubstituiert oder ortho- oder para-substituiert mit der OR"-Gruppe, wobei R" lineares oder verzweigtes substituiertes C₁-C₆-Alkyl ist, oder ein Threonin substituiert mit OR"' ist, wobei R"' H oder ein linearer verzweigter Alkylrest oder ein C₆H₅-CH₂-Rest entweder unsubstituiert oder ortho-oder para-substituiert mit der OR"-Gruppe ist, wobei R" wie vorstehend definiert oder ein substituiertes C₆H₅-CH₂- oder 1- oder 2-Naphthylmethylist; andernfalls Yaa 1-Nal oder 2-Nal als solches unsubstituiert oder an dem 1- oder 2-Ring durch -OCH₃ oder C₆H₅-CH₂-O- substituiert ist;
- wobei R1 ein C₂-C₄-Alkinylrest ist, wenn R2 ein C₁-C₄-Azidorest ist und umgekehrt,
m und n gleich oder verschieden sind und 1 bis 5 sind.

2. Konjugierte Somatostatin-Analoga gemäß Anspruch 1, wobei R1 und R2 eine intramolekulare 1,4-disubstituierte [1,2,3]-Triazolyl-Brücke T bilden, wobei die resultierenden Verbindungen eine Formel (II) aufweisen: 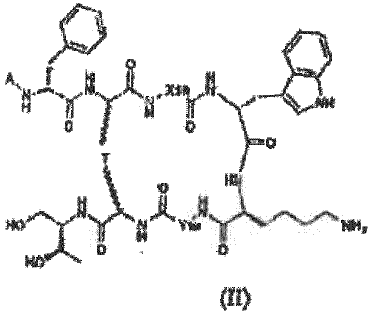 wobei A, Xaa, Yaa die gleichen wie vorstehend beschrieben sind, die Ausrichtung der 1,4-disubstituierten [1,2,3]-Triazolyl-Struktureinheit von der Position von ω-Azido oder ω-Alkinyl-Aminosäuren in der Peptidkette abhängt, jeweils i+5 und umgekehrt:

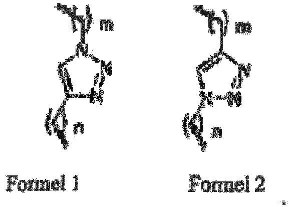

3. Konjugierte Somatostatin-Analoga gemäß einem der Ansprüche 1 oder 2, wobei
- C ausgewählt ist aus der Gruppe, umfassend 0, CH₂-, CH₂CH₂-, CH₂Ar-, CH₂CH₂Ar-, CH₂CONHCH₂CH₂-, CH₂CONHCH₂CH₂NHCH₂-, CH₂NHCOCH₂Ar-, CH₂NHCOCHArNHCOCH₂-, CH₂NHCH₂ArOCH₂-; oder
- E ausgewählt ist aus einer Gruppe, enthaltend einen Aminosäurerest, ausgewählt aus den 22 natürlichen α-Aminosäuren, β- oder γ-Aminosäureresten, oder einer Gruppe, enthaltend eine unnatürliche Aminosäure, die an α-Amino- oder Carboxygruppe und/oder in Seitenkettenposition modifiziert ist.

4. Konjugierte Somatostatin-Analoga gemäß einem der Ansprüche 1 bis 3, wobei A TAG-B darstellt.

5. Konjugierte Somatostatin-Analoga gemäß einem der Ansprüche 1 bis 4, wobei TAG-B = CA-B, das mit ¹¹¹In, ^{68/69}Ga oder ⁶⁴Cu chelatisiert ist, oder FD-B oder BA-B zur Bildgebung ist.

6. Konjugierte Somatostatin-Analoga gemäß einem der Ansprüche 1 bis 4, wobei TAG-B = CA-B ist und mit zum Beispiel ⁹⁰Y, ¹⁷⁷Lu oder ⁶⁷Cu zur Therapie chelatisiert ist.

7. Konjugierte Somatostatin-Analoga gemäß einem der Ansprüche 1 bis 4, wobei TAG ein cytotoxisches Molekül CT ist.

8. Verfahren zur Herstellung der konjugierten Somatostatin-Analoga der Formel (I) gemäß Anspruch 1 durch Festphasen-Peptid-Synthese (SPPS), umfassend:
- Verankern einer Peptidsequenz an einem derivatisierten Chlortrityl-Harz
- Wiedergewinnen des rohen Peptids durch Behandeln des Harzes zum Beispiel mit Trifluoressigsäure
- Trennen durch Fällung zum Beispiel unter Verwendung von Ethylether
- Durchführen einer sukzessiven Lyophilisierung
- Reinigen des Peptids unter Verwendung chromatographischer Technik, zum Beispiel RP-HPLC.

9. Verfahren gemäß Anspruch 8, weiterhin umfassend zur Herstellung der Derivate der Formel (II):
a) Synthetisieren eines linearen Heptapeptids folgend der Fmoc/tBu-SPPS-Strategie mit geeigneten Seitenketten-geschützten Aminosäuren,
b) Cyclisieren der mit den an das Harz gekoppelten Peptiden mittels Cu(I)-katalysierter Azid-Alkin-1,3-dipolare Huisgens-Cycloaddition zur Bildung einer regioselektiven 1,4-disubstituierten [1,2,3]-Triazolyl-Brücke
und entweder
c) Zugeben der N-terminalen Aminosäure und der Gruppe A, wie vorstehend definiert, in die Festphase oder alternativ folgend der in den Schritten c' bis e' beschriebenen Lösungsstrategie,
d) Spalten der konjugierten 1,4-disubstituierten [1,2,3]-Triazolyl-Brücke enthaltenden Peptide von dem Harz mit einer geeigneten Säure
e) Reinigen der rohen 1,4-disubstituierten [1,2,3]-Triazolyl enthaltenden cyclischen konjugierten Peptide durch halbpräparative RP-HPLC
oder
c') Zugeben der N-terminalen Aminosäuren und/oder der Gruppe E-G, wie vorstehend beschrieben definiert; Spalten des Peptids von dem Harz mit einer geeigneten Säure,
d') Koppeln der Gruppe TAG in Lösung mit den 1,4-disubstituierten [1,2,3]-Triazolyl-Brücke enthaltenden Peptiden,
e') Reinigen der rohen 1,4-disubstituierten [1,2,3]-Triazolyl-Brücke enthaltenden konjugierten Peptide durch halbpräparative RP-HPLC.

10. Verfahren nach Anspruch 8 oder 9, weiterhin umfassend, wenn A TAG-B (oder TAG-C-D-E) darstellt, Konjugieren des resultierenden Peptids mit einem TAG-Derivat der Formel (III)
TAG-C-F, (III)
wobei C und F die gleichen wie vorstehend beschrieben sind.

11. Verbindungen gemäß den Ansprüchen 1 bis 5, verwendet zur optischen Bildgebung.

12. Radioaktive Tracer gemäß Anspruch 1 bis 6, verwendet zur SPECT-/PET-Bildgebung oder zur nuklearmedizinisch/optischen Bildgebung; oder enthaltend wenigstens ein konjugiertes Somatostatin-Analogon nach einem der Ansprüche 1 bis 6 zur Bildgebung bei Tumorzellen.

13. Verfahren zur Radioisotop-Bildgebung, umfassend die Verwendung von wenigstens einer Verbindung gemäß Formel (I) oder (II), wobei A TAG-B darstellt.

14. Pharmazeutische Zusammensetzungen, enthaltend eine therapeutisch effiziente Menge von wenigstens einem konjugierten Somatostatin-Analogon der Formel (I) in Kombination mit einem pharmazeutisch annehmbaren Träger.

15. Pharmazeutische Zusammensetzungen gemäß Anspruch 14, wobei das oder die konjugierten Somatostatin-Analoga zur Behandlung von Krebs, insbesondere zur Behandlung von Lymphom, Bauchspeicherdrüsen-, Lungen-, Prostata- oder Brustkrebs oder Adenom, eingesetzt werden.

## Revendications

1. Analogues de somatostatine conjugués de
formule [I], 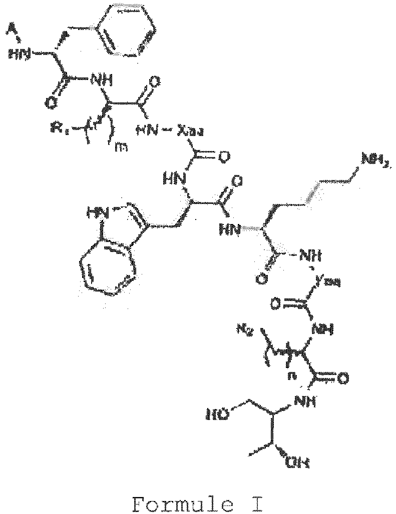
- dans laquelle A- est H ou TAG-B-,
• où -B- est 0 ou un espaceur de formule C-D-E,
∘ -C- et E sont identiques ou différents et sont :
▪ une chaine -(CH₂)ᵢW où i varie de 0 à 20 et peut être de préférence 0 et 1 et W est 0, une chaîne contenant 1 à 6 groupe(s) (C=O) ou (C=S) et/ou un ou plusieurs hétéroatomes (N, O, S, P), et/ou une à trois fraction(s) aromatique(s) contenant zéro, un ou plusieurs hétéroatomes (N, O, S, P), ortho et/ou méta et/ou para substitués ou une chaîne contenant une à 12 fractions d'acide aminé choisies parmi les 22 acides α-aminés naturels, des résidus d'acides β ou γ-aminés, de préférence la lysine ou la thréonine,
▪ une chaîne (PE_{G})ⱼ-W où j = 2, 4, 100, 300, 1 000 et W est comme décrit ci-dessus,
o D étant un groupe résultant du couplage de deux groupes fonctionnels F et G présents sur le TAG et sur le peptide respectivement et défini comme suit :
D = thiourée générée à partir de F = -NH₂ et G = -N=C=S (et vice versa), D = amide généré à partir de F = NH₂ et G = COX (X = ester activé, Cl, anhydride, ...) (et vice versa), D = thioéther généré à partir de F = maléimide- et G = -SH (et vice versa) ; D = [1,2,3]-triazole 1,4-disubstitué [généré à partir de F = -N=N⁺=N⁻ et G = -alcyne (et vice versa), D = imine générée à partir de F = aldéhyde et G = NH₂ (et vice versa), D = fonction de couplage générée à partir de F = norbornène ou trans-cyclooctène et G = 1, 2, 4, 5-tétrazine (et vice versa),
• où TAG est
- un agent chélatant CA
où CA-B est l'une des formules 1 à 8,
R et R' sont des groupes capables de chélater un métal d'intérêt, soit à des fins d'imagerie (Gd (IRM), ¹¹¹In ou ⁶⁷Ga (TEPU), ⁶⁴Cu ou ⁶⁸Ga (TEP), ...) soit de thérapie (⁹⁰Y, ¹⁷⁷Lu, ⁶⁷Cu, ...), préférentiellement COO⁻, COOH, P(O) (OH)Me ou CONH₂ 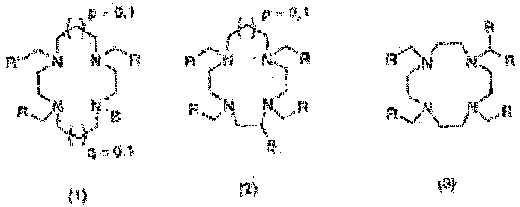 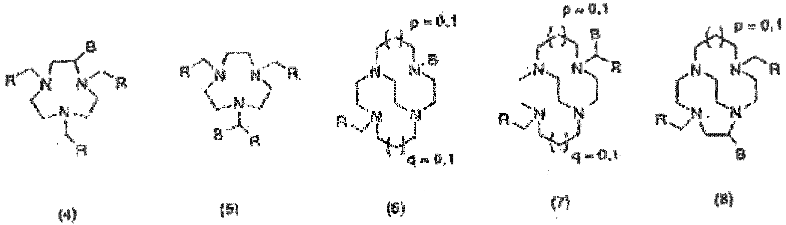
- une teinte fluorescente FD où FD-B est un fluorophore organique B tel qu'un dérivé de Bodipy B, un dérivé de rhodamine B, un dérivé de fluorescéine B, un dérivé de cyanine B, un dérivé de porphyrine B ou un complexe fluorescent de formules (9) à (12) où J est un groupe de chromophore et Ln est un lanthanide choisi parmi des lanthanides donnant des complexes de lanthanide hautement fluorescents, 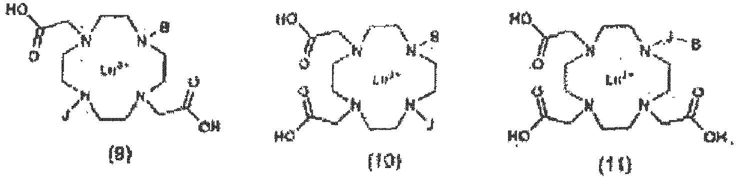 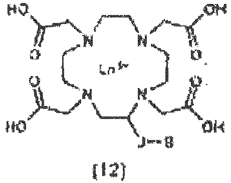
- un agent bimodal BA comprenant à la fois un agent chélatant CA et un fluorophore FD couplés ensemble en utilisant en particulier un lieur acide aminé, par exemple BA-B étant l'une de la formule 13 ou 14 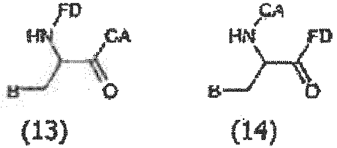 FD et B sont les mêmes que décrits précédemnent,
- une molécule cytotoxique CT où CT-B est un dérivé cytotoxique puissant de 2-pyrrolino-DOX (formule 15) ou doxorubicine (DOX) de formule (16) capable d'inhiber la croissance de diverses tumeurs, 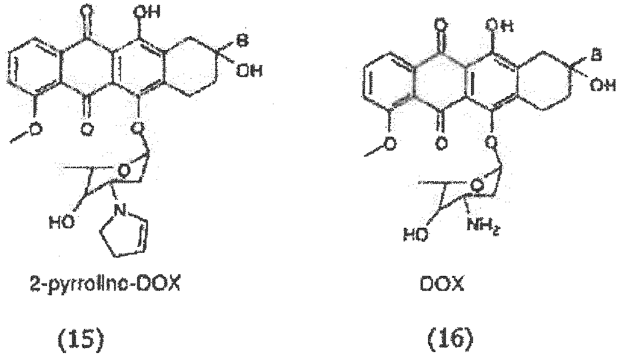
- dans laquelle les Xaa sont tous au moins l'un des 22 acides L ou D aminés ou de la phénylalanine soit non substituée, soit substituée en ortho ou *para* par le groupe OR" , où R" est substitué par alkyle en C₁ à C₆ linéaire ou ramifié ; sinon Xaa est 1-Nal (Nal = naphtylalanine) ou 2-Nal en tant que tel substitué sur le cycle 1 ou 2 par un groupe OR'' où R'' est H ou substitué par alkyle en C₁ à C₆ linéaire ou ramifié ou un C₆H₅-CH₂-,
- dans laquelle les Yaa sont tous les 22 acides L, D aminés ou de la phénylalanine soit non substituée, soit substituée en *ortho* ou *para* par le groupe OR", où R'' est substitué par alkyle en C₁ à C₆ linéaire ou ramifié ou une thréonine substituée par OR"' où R"' est H ou un résidu alkyle ramifié linéaire ou un résidu C₆H₅-CH₂- soit non substitué soit substitué en en *ortho* ou *para* par le groupe OR", où R" étant tel que défini ci-dessus ou un C₆H₅-CH₂- substitué ou 1- ou 2-naphtylméthyle- ; sinon Yaa est 1-Nal ou 2-Nal tel quel non substitué ou substitué sur le cycle 1 ou 2 par -OCH₃ ou C₆H₅-CH₂-O- ;
- dans laquelle R1 est un radical alcynyle en C₂ à C₄ lorsque R2 est un radical azido en C₁ à C₄ et vice versa,
m et n sont identiques ou différents et sont 1 à 5.

2. Analogues de somatostatine conjugués selon la revendication 1, dans lesquels R1 et R2 forment un pont T intramoléculaire [1,2,3]triazolyle 1,4-disubstitué, les composés résultants répondant à la formule (II) 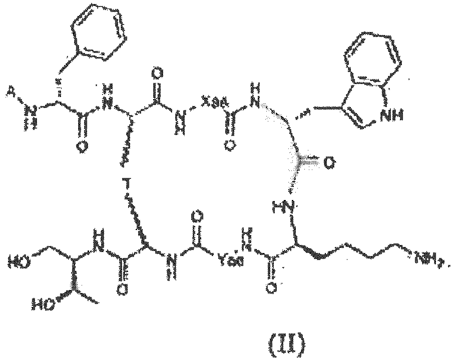 dans laquelle A, Xaa, Yaa sont les mêmes que décrits ci-dessus. L'orientation de la fraction [1,2,3]triazolyle 1,4-disubstitué dépend de la position d'acides aminés ω-azido ou ω-alcynyle dans la chaîne peptidique, respectivement I+5 et vice versa.

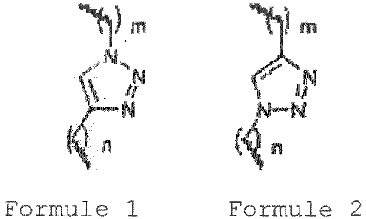

3. Analogues de somatostatine conjugués selon la revendication 1 ou 2, dans lesquels :
- C est choisi dans le groupe comprenant 0, CH₂-, CH₂CH₂-, CH₂Ar-, CH₂CH₂Ar-, CH₂CONHCH₂CH₂- ; CH₂CONHCH₂CH₂NHCH₂-, CH₂NHCOCH₂Ar-, CH₂NHCOCHArNHCOCH₂-, CH₂NHCH₂ArOCH₂- ; ou
- E est choisi dans un groupe contenant une fraction d'acide aminé choisie parmi les 22 acides α-aminés naturels, des résidus d'acides β ou γ aminés ou un groupe contenant un acide aminé non naturel modifié sur un groupe α amino ou carboxyle et/ou dans une position de chaîne latérale.

4. Analogues de somatostatine conjugués selon l'une quelconque des revendications 1 à 3, dans lesquels A représente TAG-B.

5. Analogues de somatostatine conjugués selon l'une quelconque des revendications 1 à 4, dans lesquels TAG-B est CA-B chélaté avec ¹¹¹In, ^{68/69}Ga ou ⁶⁴Cu ou FD-B ou BA-B pour imagerie.

6. Analogues de somatostatine conjugués selon l'une quelconque des revendications 1 à 4, dans lesquels TAG-B est CA-B et chélaté, par exemple, avec ⁹⁰Y, ¹⁷⁷Lu ou ⁶⁷Cu pour thérapie.

7. Analogues de somatostatine conjugués selon l'une quelconque des revendications 1 à 4, dans lesquels TAG est une molécule cytotoxique CT.

8. Procédé de préparation des analogues de somatostatine conjugués de formule (I) selon la revendication 1, par synthèse de peptides en phase solide (SPPS), comprenant les étapes consistant à
- ancrer une séquence peptidique sur une résine chlorotrityle dérivatisée
- récupérer le peptide brut en traitant la résine avec, par exemple, de l'acide trifluoroacétique
- séparer par précipitation par exemple à l'aide d'éthyléther
- réaliser une lyophilisation successive
- purifier le peptide par une technique chromatographique, par exemple RP-HPLC.

9. Procédé selon la revendication 8, comprenant en outre, pour préparer les dérivés de formule (II), les étapes consistant à
a) synthétiser un heptapeptide linéaire en suivant la stratégie SPPS Fmoc/tBu avec des acides aminés protégés en chaîne latéral appropriés,
b) cycliser les peptides liés à la résine au moyen d'une cycloaddition de Hulsgen 1,3-dipolaire azide-alcyne catalysée par Cu(I) pour former un pont [1,2,3]triazolyle 1,4-disubstitué
et soit
c) ajouter l'acide aminé à terminaison N et le groupe A défini comme décrit ci-dessus dans une phase solide ou en variante en suivant la stratégie en solution décrite dans les étapes c' à e',
d) cliver le pont [1,2,3]triazolyle 1,4-disubstitué conjugué contenant des peptides issus de la résine avec un acide approprié
e) purifier par RP-HPLC semi-préparative le [1,2,3]triazolyle 1,4-disubstitué brut contenant les peptides conjugués cycliques
soit
c') ajouter les acides aminés de la terminaison N et/ou le groupe E-G, définis comme décrits ci-dessus ; cliver le peptide issu de la résine avec un acide approprié,
d') coupler le groupe TAG en solution avec le pont [1,2,3]triazolyle 1,4-disubstitué contenant des peptides, e') purifier par RP-HPLC semi-préparative le pont [1,2,3]triazolyle 1,4-disubstitué brut contenant des peptides conjugués.

10. Procédé selon la revendication 8 ou 9, comprenant en outre lorsque A représente TAG-B (ou TAG-C-D-E), le fait de conjuguer le peptide résultant à un dérivé de tag de formule (III)
**TAG-C-F** **(III)**
dans laquelle C et F sont les mêmes que décrits ci-dessus.

11. Composés selon les revendications 1 à 5, utilisés pour une imagerie optique.

12. Radiotraceurs selon les revendications 1 à 6, utilisés pour une imagerie TEPU/TEP ou pour une imagerie nucléaire/optique ; ou comprenant au moins un analogue de somatostatine conjugué selon l'une quelconque des revendications 1 à 6 pour imager des cellules tumorales.

13. Procédé d'imagerie par radio-isotopes comprenant l'utilisation d'au moins un composé selon la formule (I) ou (II) dans laquelle A représente TAG-B.

14. Composition pharmaceutique comprenant une quantité thérapeutiquement efficace d'au moins un analogue de somatostatine conjugué de formule (I) en combinaison avec un support pharmaceutiquement acceptable.

15. Compositions pharmaceutiques selon la revendication 14, dans lesquelles le(s)dit(s) analogue(s) de somatostatine conjugué(s) est (sont) utilisé(s) pour le traitement du cancer, en particulier pour le traitement d'un lymphome, d'un cancer du pancréas, du poumon, de la prostate ou du sein, ou d'un adénome.
